# EUROPEAN PATENT SPECIFICATION

(11) **EP 2 331 114 B1**
(45) Date of publication and mention of the grant of the patent: **16.05.2012**
(21) Application number: 09782523.6
(22) Date of filing: 02.09.2009
(51) Int. Cl.: A61K 38/17

(54) **FC GAMMA RECEPTOR FOR THE TREATMENT OF B CELL MEDIATED MULTIPLE SCLEROSIS**
FC-GAMMA-REZEPTOR ZUR BEHANDLUNG VON B-ZELL-VERMITTELTER MULTIPLER SKLEROSE
RECEPTEUR FC-GAMMA POUR LE TRAITEMENT DE LA SCLÉROSE EN PLAQUES MÉDIÉE PAR LES LYMPHOCYTES B

(30) Priority: 05.09.2008 EP 08163800
(43) Date of publication of application: 15.06.2011
(73) Proprietor: SuppreMol GmbH, 82152 Martinsried/München (DE)
(72) Inventor: BUCKEL, Peter, 82347 Bernried (DE); JACOB, Uwe, 80339 München (DE)
(74) Representative: Kilger, Christian
(86) International application number: PCT/EP2009/061359
(87) International publication number: WO 2010/026168

(56) References cited:
- WO-A-2008/091682
- US-A1- 2005 175 998
- SCHAEFER B ET AL: "Sa.31. Targeting Fcgamma Receptors in the Treatment of Multiple Sclerosis" CLINICAL IMMUNOLOGY, ACADEMIC PRESS, US, vol. 119, 1 January 2006 (2006-01-01), pages S115-S116, XP024911490 ISSN: 1521-6616 [retrieved on 2006-01-01]
- TORKILDSEN O; VEDELER C A; NYLAND H I; MYHR K -M: "Fc gamma R and multiple sclerosis: an overview" ACTA NEUROLOGICA SCANDINAVICA, vol. 113, no. Suppl. 183, 2006, pages 61-63, XP009110961 ISSN: 0001-6314

## Description

### FIELD OF THE INVENTION

The invention is in the field of biotechnology and therapeutics. The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis.

### BACKGROUND OF THE INVENTION

Current hypotheses favour the concept that T cells play a pivotal role in the pathogenesis of Multiple Sclerosis (MS), which was initially based upon the observation that T cells are the predominant lymphocyte class present in MS lesions (Windhagen, et al., Cytokine, secretion of myelin basic protein reactive T cells in patients with multiple sclerosis. Journal of Neuroimmunology, 91:1-9, 1998; Hafler, D.A., et al., Oral administration of myelin induces antigen-specific TGF-beta 1 secreting cells in patients with multiple sclerosis. Annals of the New York Academy of Science, 835:120-131, 1997; Lovett-Racke, A.E., et al., Decreased dependence of myelin basic protein-reactive T cells on CD28-mediated costimulation in multiple sclerosis patients, Journal of Clinical Investigation, 101:725-730, 1998) This observation continues to be a cardinal hallmark of the disease, and is supported by a number of observations. For example, active CD4+ T helper cells bearing anti-myelin T Cell Receptors (TCRs) are present in the cerospinal fluid (CSF) of patients with MS. In addition, elevated levels of Th1-like cytokines have been detected in the CSF of patients with MS and have been correlated with worsening of the disease in some cases (Calabresi et al, Cytokine expression in cells derived from CSF of multiple sclerosis patients. Journal of Neuroimmunology, 89:198-205, 1998).

There has, however, also been evidence that B cells may be involved in the development and perpetuation of MS including: .
(1) elevated immunoglobulin levels in the CSF of MS patients (Link, H., et al., Immunoglobulins in multiple sclerosis and infections of the nervous system, Archives of Neurology, 25:326-344, 1971; Link, H., et al., Immunoglobulin class and light chain type of oligoclonal bands in CSF in multiple sclerosis determined by agarose gel electrophoresis and immunofixation. Ann Neurol, 6(2):107-110, 1979; Perez, L., et al., B cells capable of spontaneous IgG secretion in cerebrospinal fluid from patients with multiple sclerosis: dependancy on local IL-6 production. Clinical Experimental Immunology, 101:449-452,1995),
(2) oligoclonal banding in the CSF of MS patients (Link, H., et al., Immunoglobulin class and light chain type of oligoclonal bands in CSF in multiple sclerosis determined by agarose gel electrophoresis and immunofixation. Ann Neurol, 6(2):107-110, 1979),
(3) skewing of the κ:λ ratio in the CSF of MS patients (Hauser, S.L., et al., Clonally restricted B cells in peripheral blood of multiple sclerosis patients: kappa/lambda staining patterns. Annals of Neurology, 11:408-412,1982),
(4) the presence of anti-myelin antibodies in the CSF of MS patients (Sun, J. H., et al, B cell responses to myelin-oligodendrocyte glycoprotein in multiple sclerosis. Journal of Immunology, 146:1490-1495, 1991) and
(5) the demonstration that antibodies from the CSF of MS patients may contribute to the overall extent of tissue injury in these patients (Lassmann, H., et al., Experimental allergic encephalomyelitis: the balance between encephalitogenic T lymphocytes and demyelinating antibodies determines size and structure of demyelinated lesions. Acta Neuropathology, 75:566-576, 1988).

A number of publications demonstrate such B cell mediation: Bourquin, et al., The journal of Immunology, 2003, 171: 455-461; Stromnes et al. Nature Protocols, Vol. 1, No. 4, 2006: 1810-1818; Stromnes et al Nature Protocols, Vol. 1, No. 4, 2006: 1952-19160; Oliver, et al., The Journal of Immunology, 2003, 171: 462-468;

The basic role of B cells in the immune system is to mediate Humoral Immune responses. That is, to secrete proteins called antibodies (or immunoglobulins) that bind to foreign bodies and mark them for elimination from the body by other immune cells such as NK cells and macrophages.

Intravenous immune globulin (IVIG) has been demonstrated in multiple clinical trials reported in the medical literature to have an impact on two important considerations in relapsing-remitting Multiple Sclerosis. IVIG reduces the frequency of the acute exacerbation and it reduces the intensity and duration of the acute exacerbation. Seemingly, a subset of patients has a prominent B cell involvement in multiple sclerosis. Hence, it would be advantageous to have substances and methods for the treatment of such B cell mediated multiple sclerosis patients.

IVIG represents pooled human immune globulin from many donors. The exact mechanism by which IVIG ameliorates autoantibody mediated diseases is unknown. However, it could be shown that a) the efficacy of IVIG is dependant on Fc-receptor expression b) the ratio of autoantibodies to normal immune globulin (IVIG) is shifted, which leads to enhanced degradation of autoantibodies and c) the IVIG contains the complete human antibody and anti-idiotype antibody network (antibodies against antibodies) which leads to the neutralization of autoantibodies."

### SUMMARY OF THE INVENTION

The inventors have astonishingly found that the current hypothesis that T cells play a pivotal role in the pathogenesis of Multiple Sclerosis (MS) is at least not entirely correct. The inventors addressed the B cell mediated form of multiple sclerosis and are, astonishingly able to provide for a special treatment.

The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis. The invention relates to pharmaceutical compositions containing the Fcγ receptor (Fc-gamma receptor) for use in treating multiple sclerosis, wherein the multiple sclerosis is a B cell mediated form of multiple sclerosis and/or an autoantibody driven form of multiple sclerosis.

Fc receptors (FcRs) play a key role in defending the human organism against infections. After pathogens have gained access to the blood circulation they are opsonized by immunoglobulins (Igs). The resulting immunocomplexes bind due to their multivalency with high avidity to FcR bearing cells leading to clustering of the FcRs, which triggers several effector functions (Metzger, H., 1992A). These include, depending on the expressed FcR type and associated proteins, endocytosis with subsequent neutralization of the pathogens and antigen presentation, antibodydependent cellular cytotoxity (ADCC), secretion of mediators or the regulation of antibody production (Fridman, *et al.,* 1992; van de Winkel and Capel, 1993).

Specific FcRs exist for all Ig classes, the ones for IgG being the most abundant with the widest diversity. Together with the high affinity receptor for IgE (FcεRIa), FcγRI (CD64), FcγRII (CD32) and FcγRIIIa (CD16) occur as type I transmembrane proteins or in soluble forms (sFcRs) but also a glycosylphosphatidylinositol anchored form of the FcγRIII (FcγRIIIb) exists. Furthermore, FcγRs occur in various isoforms (FcγRIa, b1, b2, c; FcγRIIa1-2, b1-3, c) and alleles (FcγRIIa1-HR, -LR; FcγRIIIb-NA1 ,-NA2) (van de Winkel and Capel, 1993). In contrast to the overall homologous extracellular parts, the membrane spanning and the cytoplasmic domains differ. They may be deleted entirely or be of a size of 8 kDa. They may contain either a 26 amino acid immunoreceptor tyrosine-based activation motif (ITAM) as in FcγRIIa or a respective 13 amino acid inhibitory motif (ITIM) in FcγRIIb involved in signal transduction (Amigorena, *et al*., 1992).

Herein, EAE is experimental autoimmune encephalomyelitis.

### DETAILED DESCRIPTION OF THE INVENTION

The invention relates to the Fcγ receptor (Fc-gamma receptor) for use in treating multiple sclerosis, wherein the multiple sclerosis is a B cell mediated form of multiple sclerosis and/or an autoantibody driven form of multiple sclerosis.

The B cell mediation of the multiple sclerosis and/or autoantibody driven form of multiple sclerosis is characterized by one or more of the following features, (a) the multiple sclerosis is ameliorated If the patient undergoes Intravenous immunoglobulin (IVIG) treatment and/or, (b) the multiple sclerosis is ameliorated if the patient undergoes anti-CD20 antibody treatment and/or, (c) the multiple sclerosis is ameliorated if the patient undergoes plasmapheresis and/or, (d) the multiple Sclerosis is ameliorated if the patient undergoes immunoadsorption (e) the presence of auto-antibodies against the antigen Myelin Oligodendrocyte Glycoprotein (MOG) and/or (f) the presence of auto-antibodies against the antigen myelin basic protein (MBP) and/or (g) the presence of auto-antibodies against aquaporin 4.

Devic's disease is similar to MS in that the body's immune system attacks the myelin surrounding nerve cells. Unlike standard MS, the attacks are not believed to be mediated by the immune system's T cells but rather by antibodies called NMO-IgG. These antibodies target a protein called aquaporin 4 in the cell membranes of astrocytes which acts as a channel for the transport of water across the cell membrane.

In a preferred embodiment the B cell mediation of the multiple sclerosis and/or autoantibody driven form of multiple sclerosis is determined prior to the use of the Fcγ receptor by means of one or more of the following tests, (a) determining whether the multiple sclerosis is ameliorated if the patient undergoes Intravenous immunoglobulin (IVIG) treatment and/or, (b) the multiple sclerosis is ameliorated if the patient undergoes anti-CD20 antibody treatment and/or, (c) the multiple sclerosis is ameliorated if the patient undergoes plasmapheresis, (d) the multiple Sclerosis is ameliorated if the patient undergoes immunoadsorption (e) determining whether auto-antibodies against the antigen myelin oligodendrocyte glycoprotein (MOG) are present in the patient and/or, (f) determining whether auto-antibodies against the antigen myelin basic protein (MBP) are present in the patient and/or (g) determining the presence of auto-antibodies against aquaporin 4. Preferably a selection of two of the test are performed, more preferably a selection of three of tests are performed, more preferably a selection of four of the tests are performed.

One example of an anti-CD20 treatment is Rituximab. This is a monoclonal antibody. Sadly, it has severe side-effects which could be ameliorated if the present protein, *i.e*. polypetides were used in place of Rituximab.

During plasmapheresis, blood is initially taken out of the body through a needle or previously implanted catheter. Plasma is then removed from the blood by a cell separator. Three procedures are commonly used to separate the plasma from the blood:

### Discontinuous flow centrifugation:

One venous catheter line is required. Typically, a 300 ml batch of blood is removed at a time and centrifuged to separate plasma from blood cells.

### Continuous flow centrifugation

Two venous lines are used. This method requires slightly less blood volume to be out of the body at any one time as it is able to continuously spin out plasma.

### Plasma filtration

Two venous lines are used. The plasma is filtered using standard hemodialysis equipment. This continuous process requires less than 100 ml of blood to be outside the body at one time.

During immunoadsorption the blood of a patient is cleared from immunoglobulin by an extra corporal affinity chromatography column.

Each method has its advantages and disadvantages. After plasma separation, the blood cells are returned to the person undergoing treatment, while the plasma, which contains the antibodies, is first treated and then returned to the patient in traditional plasmapheresis. (In plasma exchange, the removed plasma is discarded and the patient receives replacement donor plasma or saline with added proteins.) Medication to keep the blood from clotting (an anticoagulant) is generally given to the patient during the procedure. Plasmapheresis is used as a therapy in particular diseases.

An important use of plasmapheresis is in the therapy of autoimmune disorders. However, the method is extremely strenuous for the patient.

WO°2008/017363 discloses means for testing for B cell mediation. In particular it discloses means of detecting auto-antibodies against MOG and aquaporin 4. WO°2008/017363 is incorporated by reference.

In a preferred embodiment of the invention the FcR receptor is of human origin. The Fcγ receptor according to the invention is preferably selected from the group of, FcγRI (CD64), FcγRIIA (CD32), FcγRIIB1 (CD32), FcγRIIB2 (CD32), FcγRIIc (CD32), FcγRIIIA (CD16) and FcγRIIIB (CD16).

FcγRIIB1 (CD32) and FcγRIIB2 (CD32) are so called isoforms, i.e. the isoforms 1 and 2.

According to the present invention, the preparation of the soluble Fc receptors takes place in prokaryotic or eukaryotic cells. It may also take place in eukaryotic cells. If it takes place in prokaryotic cells (see EP-B1 1 135 486) insoluble inclusion bodies containing the recombinant protein form, thus facilitating purification by separation of the inclusion bodies from other cell components before renaturation of the proteins contained therein takes place. The renaturation of the FcRs according to the present invention which are contained in the inclusion bodies can principally take place according to known methods. The advantage of the preparation in prokaryotic cells, the production of inclusion bodies and the thus obtained recombinant soluble Fc receptors make it possible to obtain a very pure and, in particular, also very homogeneous FcR preparation. Also because of the absence of glycosylation the obtained product is of great homogeneity. However, in some cases glycosylation may be desired.

A host cell is genetically engineered with the polynucleotide or the vector encoding or carrying the FcR. The host cells that may be used for purposes of the invention include but are not limited to prokaryotic cells such as bacteria (for example, *E*. *coli* and *B. subtilis*), which can be transformed with, for example, recombinant bacteriophage DNA, plasmid DNA, or cosmid DNA expression vectors containing the polynucleotide molecules encoding the FcR; simple eukaryotic cells like yeast (for example, *Saccharomyces* and *Pichia*), which can be transformed with, for example, recombinant yeast expression vectors containing the polynucleotide molecule of the invention, i.e. the polynucleotide molecules encoding the FcR; insect cell systems like, for example, Sf9 of Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a FcR or variant nucleotide sequence; or mammalian cell systems (for example, COS, CHO, BHK, HEK293, VERO, HeLa, MDCK, Wi38, Swiss 3T3 and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter, CMV IE and the vaccinia virus 7.5K promoter) or from bacterial cells (for example, the tet-repressor binding is employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells.

In the sequence of FcyRIIb three potential N-glycosylation sites are found. All three sites are on the surface of the molecule and are accessible. They are located in the EIF loops (N61 and N142) of both domains and on strand E (N 135) of the C-terminal domain. FcRs isolated from mammalian cells are highly glycosylated. Since FcR is glycosylated *in vivo* it may be desirable to chose an expression system, which provides faithful glycosylation of the protein. Consequently, it is preferred to introduce the polynucleotides encoding the FcR of the present invention into higher eukaryotic cells, in particular into mammalian cells, e.g. COS, CHO, BHK, HEK293, VERO, HeLa, MDCK, Wi38, Swiss 3T3 or NIH 3T3 cells.

Preferably the Fcγ receptor according to the invention lacks the transmebrane domain and/or the signal peptide and is soluble. Soluble forms of Fc receptors (sFcR) such as FcγRIII mediate isotype-specific regulation of B cell growth and immunoglobulin production. In a murine model of myeloma, sFcR suppresses growth and immunoglobulin production of tumor cells (Muller, *et al.,* 1985; Roman, *et al.,* 1988; Teillaud, *et al*., 1990). Furthermore, sFcR binds to surface IgG on cultures of human IgG-secreting myeloma cells and effects suppression of tumor cell growth and IgG secretion. Prolonged exposure of these cells to sFcR results in tumor cell cytolysis (Hoover, *et al.,* 1995).

The Fcγ receptor polypeptides can be any of those described above but with not more than ten (e.g., not more than: ten, nine, eight, seven, six, five, four, three, two, or one) conservative substitutions. Conservative substitutions are known in the art and typically include substitution of, e.g. one polar amino acid with another polar amino acid and one acidic amino acid with another acidic amino acid. Accordingly, conservative substitutions preferably include substitutions within the following groups of amino acids: glycine, alanine, valine, proline, isoleucine, and leucine (non polar, aliphatic side chain); aspartic acid and glutamic acid (negatively charged side chain); asparagine, glutamine, methionine, cysteine, serine and threonine (polar uncharged side chain); lysine, histidine and arginine; and phenylalanine, tryptophane and tyrosine (aromatic side chain); and lysine, arginine an histidine (positively charged side chain). It is well known in the art how to determine the effect of a given substitution, e.g. on pKI etc. All that is required of a polypeptide having one or more conservative substitutions is that it has at least 50% (e.g., at least: 55%; 60%; 65%, 70%; 75%; 80%; 85%; 90%; 95%; 98%; 99%; 99.5%; or 100% or more) of the activity of the unaltered Fcγ receptor according to the invention.

Both polypeptides and peptides can be produced by standard *in vitro* recombinant DNA techniques and *in vivo* transgenesis, using nucleotide sequences encoding the appropriate polypeptides or peptides. Methods well-known to those skilled in the art can be used to construct expression vectors containing relevant coding sequences and appropriate transcriptional/translational control signals. See, for example, the techniques described in Sambrook, et al., Molecular Cloning: A Laboratory Manual (2nd Ed.) [Cold Spring Harbor Laboratory, N.Y., 1989], and Ausubel, et al., Current Protocols in Molecular Biology [Green Publishing Associates and Wiley Interscience, N.Y., 1989].

Polypeptides and fragments of the invention, *i.e*. isolated polypeptides, also include those described above, but modified for *in vivo* use by the addition, at the amino-and/or carboxyl-terminal ends, of blocking agents to facilitate survival of the relevant polypeptide in vivo. This can be useful in those situations in which the peptide termini tend to be degraded by proteases prior to cellular up-take. Such blocking agents can include, without limitation, additional related or unrelated peptide sequences that can be attached to the amino and/or carboxyl terminal residues of the peptide to be administered. This can be done either chemically during the synthesis of the peptide or by recombinant DNA technology by methods familiar to artisans of average skill.

Alternatively, blocking agents such as pyroglutamic acid or other molecules known in the art can be attached to the amino and/or carboxyl terminal residues, or the amino group at the amino terminus or carboxyl group at the carboxyl terminus can be replaced with a different moiety. Likewise, the peptides can be covalently or noncovalently coupled to pharmaceutically acceptable "carrier" proteins prior to administration.

The term "isolated" polypeptide or peptide fragment as used herein refers to a polypeptide or a peptide fragment which either has no naturally-occurring counterpart or has been separated or purified from components which naturally accompany it, e.g., in tissues such as tongue, pancreas, liver, spleen, ovary, testis, muscle, joint tissue, neural tissue, gastrointestinal tissue or tumor tissue, or body fluids such as blood, serum, or urine. Typically, the polypeptide or peptide fragment is considered "isolated" when it is at least 70%, by dry weight, free from the proteins and other naturally-occurring organic molecules with which it is naturally associated. Preferably, a preparation of a polypeptide (or peptide fragment thereof) of the invention is at least 80%, more preferably at least 90%, and most preferably at least 99%, by dry weight, the polypeptide (or the peptide fragment thereof), respectively, of the invention. Thus, for example, a preparation of polypeptide x is at least 80%, more preferably at least 90%, and most preferably at least 99%, by dry weight, polypeptide x. Since a polypeptide that is chemically synthesized is, by its nature, separated from the components that naturally accompany it, the synthetic polypeptide is "isolated."

An isolated polypeptide (or peptide fragment) of the invention can be obtained, for example, by extraction from a natural source (e.g., from tissues or bodily fluids); by expression of a recombinant nucleic acid encoding the polypeptide; or by chemical synthesis. A polypeptide that is produced in a cellular system different from the source from which it naturally originates is "isolated," because it will necessarily be free of components which naturally accompany it. The degree of isolation or purity can be measured by any appropriate method, e.g., column chromatography, polyacrylamide gel electrophoresis, or HPLC analysis.

The Fcγ receptor according to the invention may be chemically modified (improved) by PEGylation and/or genetic engineering.

Known approaches involve the provision of additional glycosylation sites (see e.g. WO 91/05867, WO 94/09257 and WO 01/81405). Such modified analogs may have at least one additional N-linked and/or O-linked carbohydrate chain. Other attempts to improve the half life may involve the addition of polyethylene glycol residues (PEG) of varying length to the amino acid backbone (see e.g. WO 00/32772, WO 01/02017, WO 03/029291). One may modify the molecules with at least one N-linked and/or O-linked oligosaccharide which are further modified by oxidation, sulfation, phosphorylation, PEGylation or a combination thereof (see WO 2005/025606). All these approaches can equally be employed to extend the half life of the variants of the present invention and accordingly in a preferred embodiment of the Fcγ receptor according to the invention the modification is selected from the group consisting of oxidation, sulfation, phosphorylation, addition of oligosaccharides or combinations thereof. If the addition of further N-linked or O-linked oligonucleotides is desired it is possible to introduce them by introducing additional glycosylation sites. It also preferred that the protein is affinity modulated.

In the practice of one aspect of the present invention, a pharmaceutical composition comprising the receptor of the invention may be administered to a mammal by any route which provides a sufficient level of activity. It can be administered systemically or locally. Such administration may be parenterally, transmucosally, e.g., orally, nasally, rectally, intravaginally, sub-lingually, submucosally or transdermally. Preferably, administration is parenteral, e.g. via intravenous or intraperitoneal injection, and also including, but is not limited to, intraarterial, intramuscular, intradermal and subcutaneous administration. If the pharmaceutical composition of the present invention is administered locally it can be injected directly into the organ or tissue to be treated. In cases of treating the nervous system this administration route includes, but is not limited to, the intracerebral, intraventricular, intracerebroventricular, intrathecal, intracisternal, intraspinal and/or peri-spinal routes of administration, which can employ intracranial and intravertebral needles, and catheters with or without pump devices.

The receptor may also by glycosylated.

Most preferably the receptor is selected from the group of FcγRIIA/B/C (CD32) and FcγRIIIA/B (CD16b). The invention also relates to isoforms thereof and isoforms of the FcRs claimed herein in general.

In a preferred embodiment of pharmaceutical composition comprises an FcR receptor polypeptide in a dosage unit form for treating multiple sclerosis, wherein the multiple sclerosis is a B cell mediated form of multiple sclerosis and/or an autoantibody driven form of multiple sclerosis, and the amount to administered to a patient in a single dose is between 1 and 20 mg/kg, preferably 2 and 10 mg/kg, more preferably between 25 and 5 mg/kg, even more preferably between 2,5 and 5 mg/kg.

A pharmaceutical composition may additionally comprise one or more of the following substances, a detergent and/or a sugar. A preferred detergent is Tween 20. A preferred sugar is manitol.

The Fcγ receptor according to the invention preferably has sequence selected from the group of, SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12 and SEQ ID NO. 13. These are outlined in Table 1.

The Fcγ receptor according to the invention preferably comprises only the extra cellular domain of said sequences (soluble form of the receptor). Said domain is known from sequence alignments, is structurally characterized by x-ray crystallography and comprise the first two (CD16, CD32) or the first three (CD64) IgG-like domains of the mature receptor (Sondermann P., Kaiser J., Jacob U., Molecular basis for immune complex recognition: a comparison of Fc-receptor structures. J. Mol. Biol. 2001, 309, 737-749).

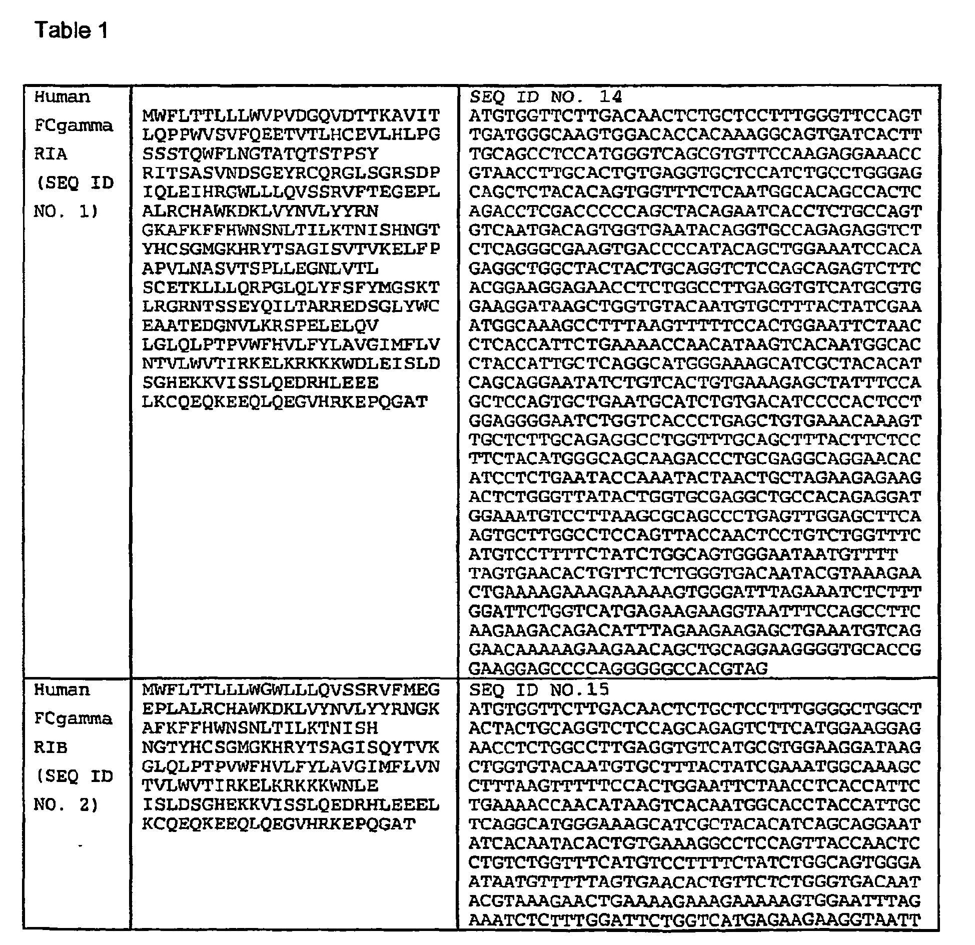

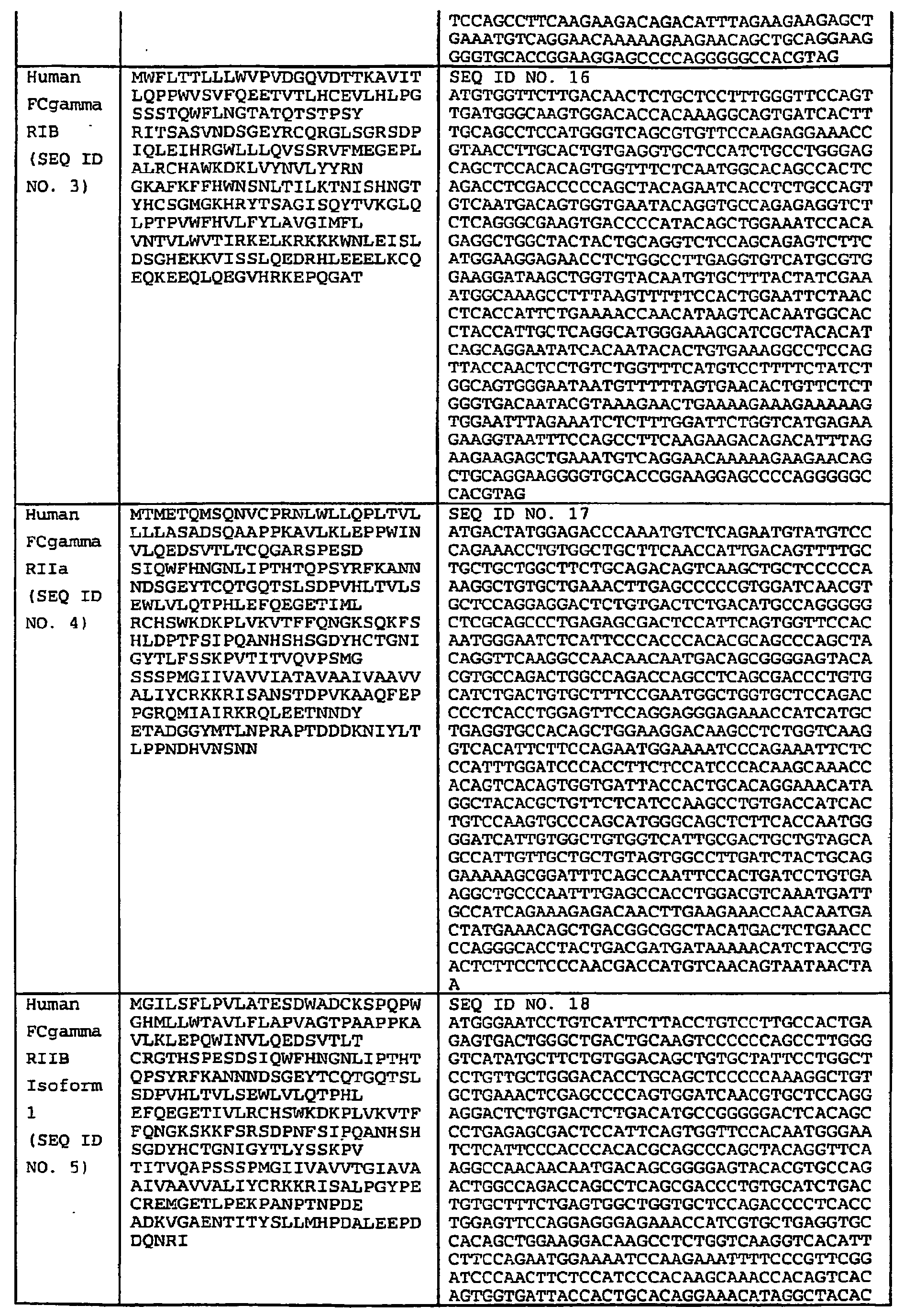

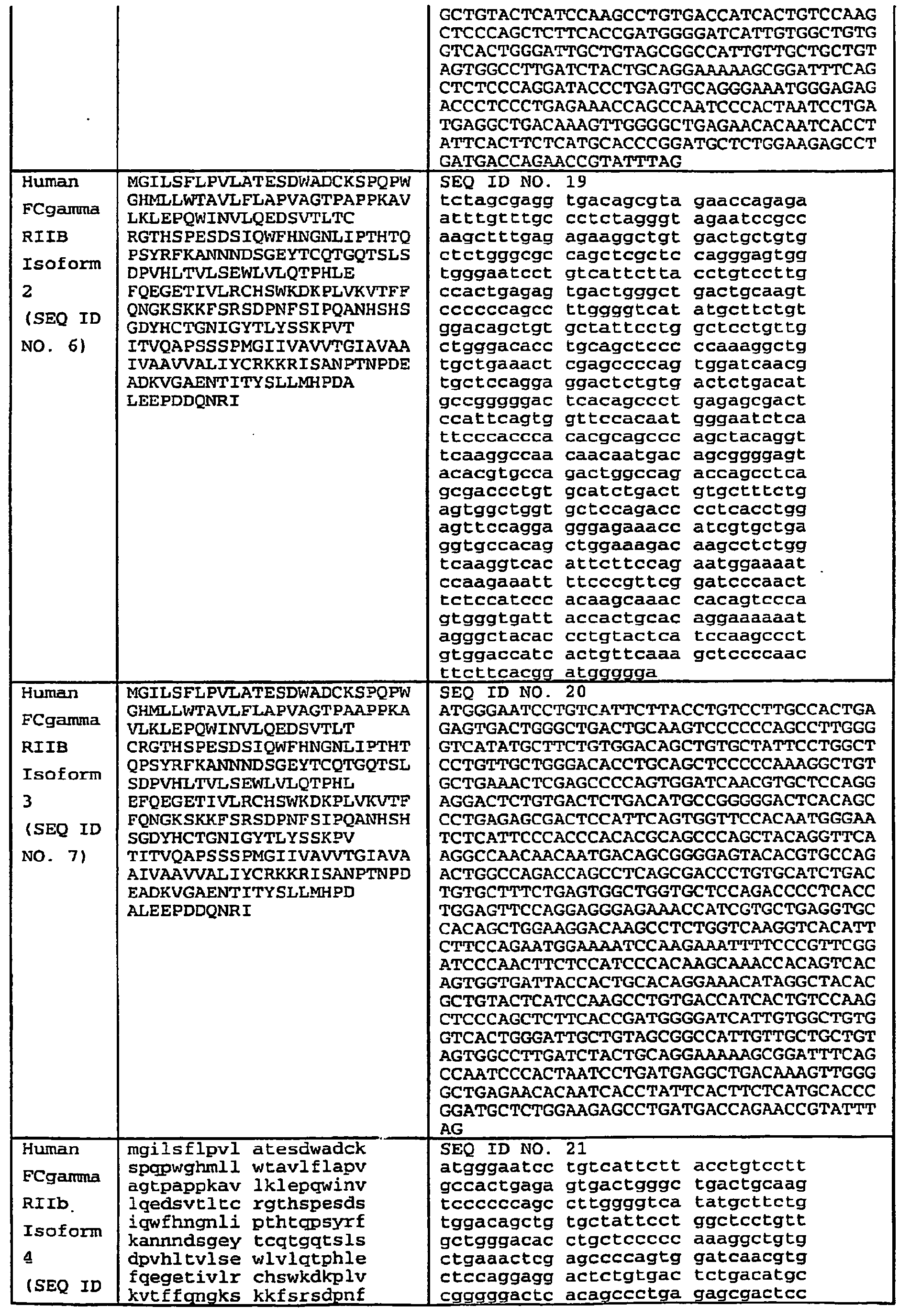

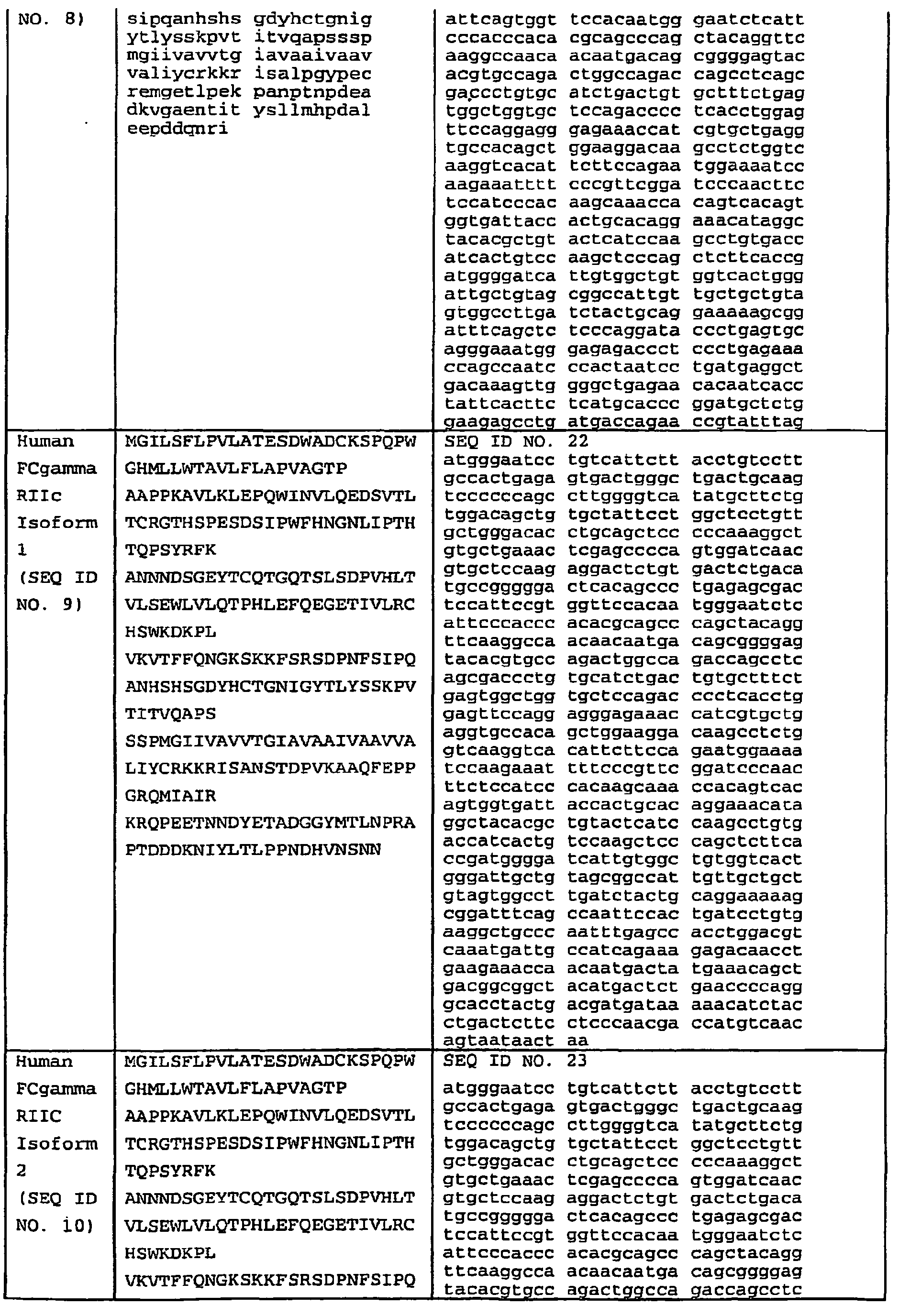

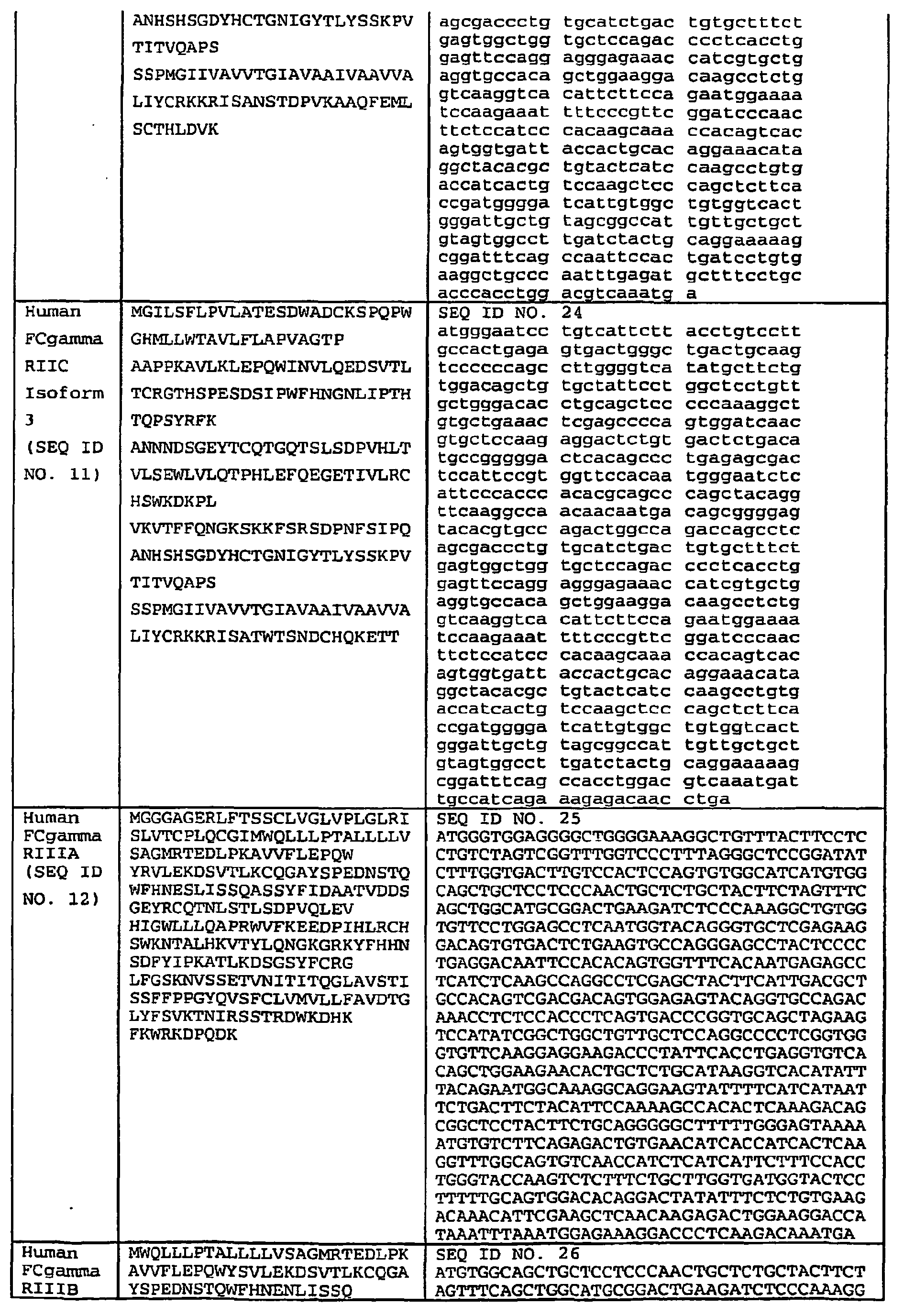

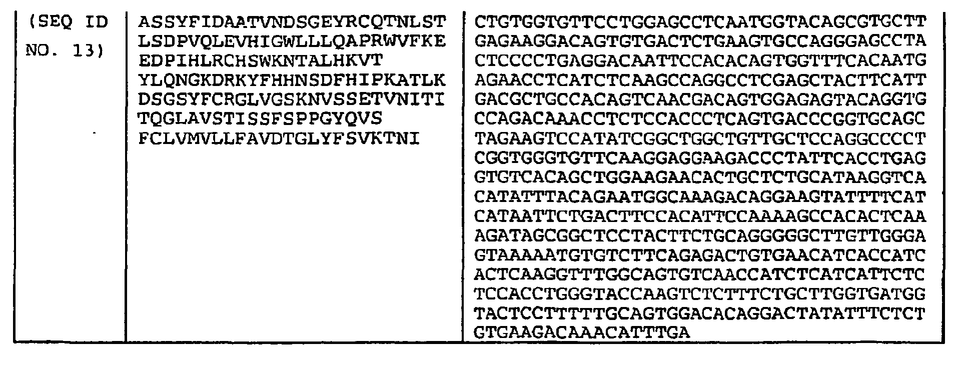

The preferred FcRs are encoded by the following nucleic acids:
Human FCgammaRIA (SEQ ID NO. 1) is encoded by the sequence according to SEQ ID NO. 14.
Human FCgammaRIB (SEQ ID NO. 2) is encoded by the sequence according to SEQ ID NO. 15.
Human FCgammaRIB (SEQ ID NO. 3) is encoded by the sequence according to SEQ ID NO. 16.
Human FCgammaRIIa (SEQ ID NO. 4) is encoded by the sequence according to SEQ ID NO. 17.
Human FCgammARIIB Isoform 1 (SEQ ID NO. 5) is encoded by the sequence according to SEQ ID NO. 18.
Human FCgammaRIIB Isoform 2 (SEQ ID NO. 6) is encoded by the sequence according to SEQ ID NO. 19.
Human FCgammaRIIB Isoform 3 (SEQ ID NO. 7) is encoded by the sequence according to SEQ ID NO. 20.
Human FCgammaRIIb Isoform 4 (SEQ ID NO. 8) is encoded by the sequence according to SEQ ID NO. 21.
Human FCgammaRIIc Isoform 1 (SEQ ID NO. 9) is encoded by the sequence according to SEQ ID NO. 22.
Human FCgammaRIIC Isoform 2 (SEQ ID NO. 10) is encoded by the sequence according to SEQ ID NO. 23.
Human FCgammaRIIC Isoform 3 (SEQ ID NO. 11) is encoded by the sequence according to SEQ ID NO. 24.
Human FCgammaRIIIA (SEQ ID NO. 12) is encoded by the sequence according to SEQ ID NO. 25.
Human FCgammaRIIIB (SEQ ID NO. 13) is encoded by the sequence according to SEQ ID NO. 26.

It is preferred that the FcγR is a recombinant non-glycosylated human soluble FcγRIIb preferably selected from the group of SEQ ID NO. 5, SEQ ID NO. 6, SEQ ID NO. 7 and SEQ ID NO. 8.

### EXAMPLES

The biologically active compound, the recombinant human soluble FcγRIIb molecule, is produced in inclusion bodies by fermentation of genetically engineered *E*. *coli.*

sFcγRIIb is produced by fermentation in *E*. *coli* strain BL21 (DE3) which has been transformed with an optimized cDNA sequence for the expression of sFcγRIIb.

The sFcγRIIb contains 4 cysteins to form 2 intra-molecular disulfide bonds between positions Cys26-Cys68 and Cys107-Cys151. The N-terminal sequence corresponds to the consensus sequence of the eukaryotic signal peptidase. The C-terminus was generated by introduction of a stop codon after the SER-SER-PRO motive. No further modifications were introduced.

The sFcγRIIb has a molecular mass of 19,688.9 at native and of 19,692.9 at reducing conditions.

An overview of the manufacturing process of the drug substance is given in Table 2:

**Table 2**

| |
|---|
| One vial of WCB |
| Fermentation of *E*. *coli* cell substrate |
| Harvest of *E*. *coli* cell substrate |
| Isolation of inclusion bodies |
| Refolding of sFcγRIIb from inclusion bodies |
| Purification of sFcγRIIb to yield bulk material |
| Storage of sFcγRIIb bulk at -80°C |

### FIGURE CAPTIONS

### Fig. 1

EAE was induced on day zero in 6-8 week old C57/Bl6j female mice by subcutaneous injection of 100 µg rat MOG in complete Freund adjuvant (CFA). 250ng of pertussistoxin were given intraperitoneally on day 0 and day 2. The mice were treated intraperitoneally with 200 µg sFcR after first symptoms appeared on day 8, 11 and 14. The scoring scheme was according to the degree of paralysis: 0=no paralysis, 1=tail paralysis, 2=hind legs paralysis, 3=front legs paralysis, 4=complete paralysis, 5=death.

### Fig. 2

EAE was induced on day zero in 6-8 week old SJL female mice by subcutaneous injection of 100 µg rat MOG in complete Freund adjuvant (CFA). 250ng of pertussistoxin were given intraperitoneally on day 0 and day 2. The mice were treated intraperitoneally with 200 µg sFcR after first symptoms appeared on day 9, 12 and 15. The scoring scheme was according to the degree of paralysis: 0=no paralysis, 1=tail paralysis, 2=hind legs paralysis, 3=front legs paralysis, 4=complete paralysis, 5=death.

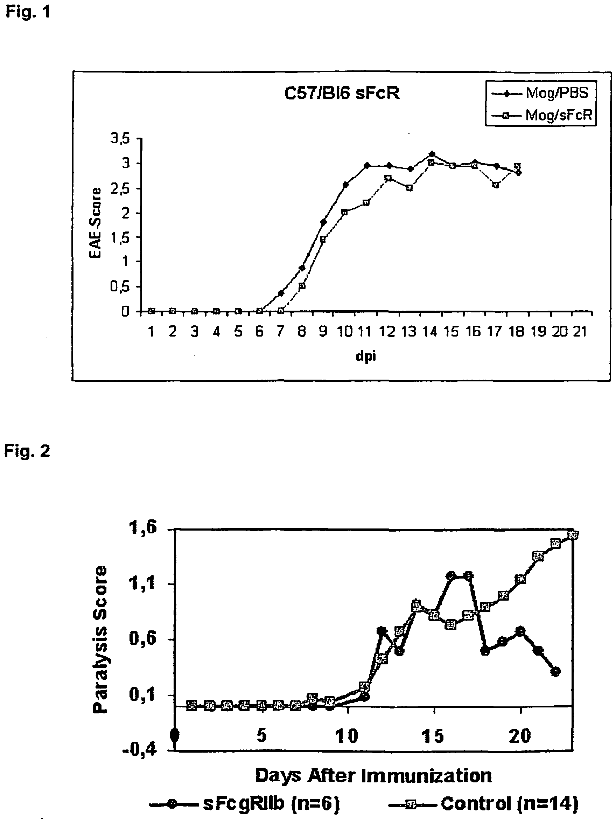

## Claims

1. Fcγ receptor (Fc-gamma receptor) for use in treating multiple sclerosis, wherein the multiple sclerosis is a B cell mediated form of multiple sclerosis and/or an autoantibody driven form of multiple sclerosis.

2. Fcγ receptor for use according to claim 1, wherein the B cell mediation of the multiple sclerosis and/or autoantibody driven form of multiple sclerosis Is **characterized by** one or more of the following features,
a. the multiple sclerosis is ameliorated if the patient undergoes Intravenous Immunoglobulin (IVIG) treatment and/or,
b. the multiple sclerosis is ameliorated if the patient undergoes anti-CD20 antibody treatment and/or,
c. the multiple sclerosis is ameliorated if the patient undergoes plasmapheresis and/or,
d. the multiple Sclerosis is ameliorated if the patient undergoes immunoadsorption
e. the presence of auto-antibodies against the antigen Myelin Oligodendrocyte Glycoprotein (MOG) and/or,
f. the presence of auto-antibodies against the antigen myelin basic protein (MBP) and/or,
g. the presence of auto-antibodies against aquaporin 4.

3. Fcγ receptor for use according to claims 1 or 2, wherein the B cell mediation of the multiple sclerosis and/or autoantibody driven form of multiple sclerosis is determined prior to the use of the Fcγ receptor by means of one or more of the following tests,
a. determining whether the multiple sclerosis is ameliorated if the patient undergoes Intravenous Immunoglobulin (IVIG) treatment and/or,
b. the multiple sclerosis is ameliorated if the patient undergoes anti-CD20 antibody treatment and/or,
c. the multiple sclerosis is ameliorated if the patient undergoes plasmapheresis and/or,
d. the multiple Sclerosis is ameliorated if the patient undergoes immunoadsorption
e. determining whether auto-antibodies against the antigen Myelin Oligodendrocyte Glycoprotein (MOG) are present in the patient and/or,
f. determining whether auto-antibodies against the antigen myelin basic protein (MBP) are present in the patient and/or,
g. determining whether auto-antibodies against aquaporin 4 are present in the patient.

4. Fcγ receptor for use according to claims 1 to 3, wherein the FcγR is selected from the group of, FcγRI (CD64), FcγRIIA (CD32), FcγRIIB1 (CD32), FcγRIIB2 (CD32), FcγRIIc (CD32), FcγRIIIA (CD16) and FcγRIIIB (CD16).

5. Fcγ receptor for use according to claims 1 to 4, wherein the receptor lacks the transmebrane domain and/or the signal peptide and is soluble.

6. Fcγ receptor for use according to claims 1 to 5, wherein the receptor is chemically modified by PEGylation and/or affinity modulated.

7. Fcγ receptor for use according to claims 1 to 6, wherein the receptor is non glycosylated.

8. Fcγ receptor for use according to claims 1 to 7, wherein the receptor is FcγRIIB/C (CD32) or FcγRIIIA/B (CD16b).

9. Fcγ receptor for use according to claims 1 to 8, in an aqueous solution, wherein the amount to administered to a patient in a single dose is between 1 and 20 mg/kg.

10. Fcγ receptor for use according to claims 1 to 9, wherein the receptor has sequence selected from the group of, SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12 and SEQ ID NO. 13.

11. Fcγ receptor for use according to claims 1 to 10, wherein the FcγR is a recombinant non-glycosylated human soluble FcγRIIb preferably selected from the group of SEQ ID NO. 5, SEQ ID NO. 6, SEQ ID NO. 7 and SEQ ID NO. 8.

## Patentansprüche

1. Fcγ-Rezeptor (Fc-gamma-Rezeptor) zur Verwendung in der Behandlung von Multipler Sklerose, wobei die Multiple Sklerose eine B-Zell-vermittelte Form von Multipler Sklerose und/oder eine durch Autoantikörper ausgelöste Form von Multipler Sklerose ist.

2. Fcγ-Rezeptor zur Verwendung gemäß Anspruch 1, wobei die B-Zell-Vermittlung der Multiplen Sklerose und/oder durch Autoantikörper ausgelöste Form von Multipler Sklerose durch eines oder mehrere der folgenden Merkmale gekennzeichnet ist,
a. die Multiple Sklerose bessert sich, wenn der Patient einer intravenösen Immunglobulinbehandlung unterzogen wird, und/oder
b. die Multiple Sklerose bessert sich, wenn der Patient einer Anti-CD20-Antikörperbehandlung unterzogen wird, und/oder
c. die Multiple Sklerose bessert sich, wenn der Patient einer Plasmapherese unterzogen wird, und/oder
d. die Multiple Sklerose bessert sich, wenn der Patient einer Immunadsorption unterzogen wird,
e. das Vorliegen von Autoantikörpern gegen das Antigen Myelin-Oligodendrozyten-Glycoprotein (MOG) und/oder
f. das Vorliegen von Autoantikörpern gegen das Antigen Myelinbasisches Protein (MBP) und/oder
g. das Vorliegen von Autoantikörpern gegen Aquaporin 4.

3. Fcγ-Rezeptor zur Verwendung gemäß Anspruch 1 oder 2, wobei die B-Zell-Vermittlung der Multiplen Sklerose und/oder die durch Autoantikörper ausgelöste Form der Multiplen Sklerose vor der Verwendung des Fcy-Rezeptors durch einen oder mehrere der folgenden Tests bestimmt wird,
a. Bestimmen, ob die Multiple Sklerose sich bessert, wenn der Patient einer intravenösen Immunglobulinbehandlung unterzogen wird, und/oder
b. die Multiple Sklerose sich bessert, wenn der Patient einer Anti-CD20-Antikörperbehandlung unterzogen wird, und/oder
c. die Multiple Sklerose sich bessert, wenn der Patient einer Plasmapherese unterzogen wird, und/oder
d. die Multiple Sklerose sich bessert, wenn der Patient einer Immunadsorption unterzogen wird,
e. Bestimmen, ob Autoantikörper gegen das Antigen Myelin-Oligodendrozyten-Glycoprotein (MOG) im Patienten vorliegen, und/oder
f. Bestimmen, ob Autoantikörper gegen das Antigen Myelinbasisches Protein (MBP) im Patienten vorliegen, und/oder
g. Bestimmen, ob Autoantikörper gegen Aquaporin 4 im Patienten vorliegen.

4. Fcγ-Rezeptor zur Verwendung gemäß den Ansprüchen 1 bis 3, wobei der FcγR ausgewählt ist aus der Gruppe von FcγRI (CD64), FcγRIIA (CD32), FcγRIIB1 (CD32), FcγRIIB2 (CD32), FcγRIIc (CD32), FcγRIIIA (CD16) und FcγRIIIB (CD16).

5. Fcγ-Rezeptor zur Verwendung gemäß den Ansprüchen 1 bis 4, wobei dem Rezeptor die Transmembrandomäne und/oder das Signalpeptid fehlt und er löslich ist.

6. Fcγ-Rezeptor zur Verwendung gemäß den Ansprüchen 1 bis 5, wobei der Rezeptor mittels PEGylierung chemisch modifiziert und/oder affinitätsmoduliert ist.

7. Fcγ-Rezeptor zur Verwendung gemäß den Ansprüchen 1 bis 6, wobei der Rezeptor nicht glykosyliert ist.

8. Fcγ-Rezeptor zur Verwendung gemäß den Ansprüchen 1 bis 7, wobei der Rezeptor FcγRIIB/C (CD32) oder FcγRIIIA/B (CD16b) ist.

9. Fcγ-Rezeptor zur Verwendung gemäß den Ansprüchen 1 bis 8 in wässriger Lösung, wobei die Menge, die einem Patienten in einer einzelnen Dosis verabreicht werden soll, zwischen 1 und 20 mg/kg ist.

10. Fcγ-Rezeptor zur Verwendung gemäß den Ansprüchen 1 bis 9, wobei der Rezeptor die Sequenz ausgewählt aus der Gruppe von SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, SEQ ID NO. 10, SEQ ID NO. 11, SEQ ID NO. 12 und SEQ ID NO. 13 hat.

11. Fcγ-Rezeptor zur Verwendung gemäß den Ansprüchen 1 bis 10, wobei der FcγR ein rekombinanter nichtglykolisierter menschlicher löslicher FcγRIIb ist, vorzugsweise ausgewählt aus der Gruppe von SEQ ID NO. 5, SEQ ID NO. 6, SEQ ID NO. 7 und SEQ ID NO. 8.

## Revendications

1. Récepteur Fcγ (récepteur Fc-gamma) pour l'utilisation dans le traitement de la sclérose en plaques, dans lequel la sclérose en plaques est une forme de la sclérose en plaques induite par des lymphocytes B et/ou une forme de sclérose en plaques entraînée par un auto-anticorps.

2. Récepteur Fcγ pour l'utilisation selon la revendication 1, dans lequel l'induction de la sclérose en plaques par les lymphocytes B et/ou la forme de sclérose en plaques entraînée par un auto-anticorps est **caractérisé par** l'une ou plusieurs des caractéristiques suivantes :
a. la sclérose en plaques est améliorée si le patient subit un traitement par immunoglobulines intraveineuses (IVIG) et/ou
b. la sclérose en plaques est améliorée si le patient subit un traitement par anticorps anti-CD20 et/ou
c. la sclérose en plaques est améliorée si le patient subit une plasmaphérèse et/ou,
d. la sclérose en plaques est améliorée si le patient subit une immuno-adsorption,
e. la présence d'auto-anticorps contre l'antigène de la glycoprotéine myéline oligodendrocyte (MOG) et/ou
f. la présence d'auto-anticorps contre la protéine basique de la myéline antigénique (MBP) et/ou
g. la présence d'auto-anticorps contre l'aquaporine 4.

3. Récepteur Fcγ pour l'utilisation selon la revendication 1 ou 2, dans lequel l'induction de la sclérose en plaques par les lymphocytes B et/ou la forme de sclérose en plaques entraînée par un auto-anticorps est déterminée avant l'utilisation du récepteur Fcγ au moyen d'un ou plusieurs des tests suivants :
a. la détermination du fait que la sclérose en plaques est améliorée ou non si le patient subit un traitement par immunoglobulines intraveineuses (IVIG) et/ou
b. la sclérose en plaques est améliorée si le patient subit un traitement par anticorps anti-CD20 et/ou
c. la sclérose en plaques est améliorée si le patient subit une plasmaphérèse et/ou,
d. la sclérose en plaques est améliorée si le patient subit une immuno-adsorption,
e. la détermination du fait que les auto-anticorps contre l'antigène de la glycoprotéine myéline oligodendrocyte (MOG) sont présents ou non chez le patient et/ou
f. la détermination du fait que des auto-anticorps contre la protéine basique de la myéline antigénique (MBP) sont présents ou non chez le patient et/ou
g. la détermination du fait que des auto-anticorps contre l'aquaporine 4 sont présents ou non chez le patient.

4. Récepteur Fcγ pour l'utilisation selon les revendications 1 à 3, le FcγR étant choisi dans le groupe comprenant FcγRI (CD64), FcγRIIA (CD32), FcγRIIB1 (CD32), FcγRIIB2 (CD32), FcγRIIc (CD32), FcγRIIIA (CD16) et FcγRIIIB (CD16).

5. Récepteur Fcγ pour l'utilisation selon les revendications 1 à 4, le récepteur étant dépourvu du domaine transmembranaire et/ou du peptide de signal et est soluble.

6. Récepteur Fcγ pour l'utilisation selon les revendications 1 à 5, le récepteur étant modifié chimiquement par PEGylation et/ou modulé par affinité.

7. Récepteur Fcγ pour l'utilisation selon les revendications 1 à 6, le récepteur étant non glycosylé.

8. Récepteur Fcγ pour l'utilisation selon les revendications 1 à 7, le récepteur étant FcγRIIB/C (CD32) ou FcγRIIIA/B (CD16b).

9. Récepteur Fcγ pour l'utilisation selon les revendications 1 à 8, dans une solution aqueuse, la quantité à administrée à un patient en une dose unique étant entre 1 et 20 mg/kg.

10. Récepteur Fcγ pour l'utilisation selon les revendications 1 à 9, le récepteur ayant une séquence choisie dans le groupe comprenant SEQ ID N° : 1, SEQ ID N° : 2, SEQ ID N° : 3, SEQ ID N° : 4, SEQ ID N° : 5, SEQ ID N° : 6, SEQ ID N° : 7, SEQ ID N° : 8, SEQ ID N° : 9, SEQ ID N° : 10, SEQ ID N° : 11, SEQ ID N° : 12 et SEQ ID N° : 13.

11. Récepteur Fcγ pour l'utilisation selon les revendications 1 à 10, FcγR étant un FcγRIIb humain soluble recombinant, non glycosylé, choisi de préférence dans le groupe SEQ ID N° : 5, SEQ ID N° : 6, SEQ ID N° : 7 et SEQ ID N° : 8.


### SEQUENCE LISTING

<110> SuppreMol GmbH
<120> SUBSTANCES AND METHODS FOR THE TREATMENT OF B CELL MEDIATED MULTIPLE SCLEROSIS
<130> P1673 PCT BLN
<160> 26
<170> PatentIn version 3.3
<210> 1
   <211> 374
   <212> PRT
   <213> Homo sapiens
<400> 1
   Met Trp Phe Leu Thr Thr Leu Leu Leu Trp Val Pro Val Asp Gly Gln
   1               5                   10                  15
   Val Asp Thr Thr Lys Ala Val Ile Thr Leu Gln Pro Pro Trp Val Ser
               20                  25                  30
   Val Phe Gln Glu Glu Thr Val Thr Leu His Cys Glu Val Leu His Leu
           35                  40                  45
   Pro Gly Ser Ser Ser Thr Gln Trp Phe Leu Asn Gly Thr Ala Thr Gln
       50                  55                  60
   Thr Ser Thr Pro Ser Tyr Arg Ile Thr Ser Ala Ser Val Asn Asp Ser
   65                  70                  75                  80
   Gly Glu Tyr Arg Cys Gln Arg Gly Leu Ser Gly Arg Ser Asp Pro Ile
                   85                  90                  95
   Gln Leu Glu Ile His Arg Gly Trp Leu Leu Leu Gln Val Ser Ser Arg
               100                 105                 110
   Val Phe Thr Glu Gly Glu Pro Leu Ala Leu Arg Cys His Ala Trp Lys
           115                 120                 125
   Asp Lys Leu Val Tyr Asn Val Leu Tyr Tyr Arg Asn Gly Lys Ala Phe
       130                 135                 140
   Lys Phe Phe His Trp Asn Ser Asn Leu Thr Ile Leu Lys Thr Asn Ile
   145                 150                 155                 160
   Ser His Asn Gly Thr Tyr His Cys Ser Gly Met Gly Lys His Arg Tyr
                   165                 170                 175
   Thr Ser Ala Gly Ile Ser Val Thr Val Lys Glu Leu Phe Pro Ala Pro
               180                 185                 190
   Val Leu Asn Ala Ser Val Thr Ser Pro Leu Leu Glu Gly Asn Leu Val
           195                 200                 205
   Thr Leu Ser Cys Glu Thr Lys Leu Leu Leu Gln Arg Pro Gly Leu Gln
       210                 215                 220
   Leu Tyr Phe Ser Phe Tyr Met Gly Ser Lys Thr Leu Arg Gly Arg Asn
   225                 230                 235                 240
   Thr Ser Ser Glu Tyr Gln Ile Leu Thr Ala Arg Arg Glu Asp Ser Gly
                   245                 250                 255
   Leu Tyr Trp Cys Glu Ala Ala Thr Glu Asp Gly Asn Val Leu Lys Arg
               260                 265                 270
   Ser Pro Glu Leu Glu Leu Gln Val Leu Gly Leu Gln Leu Pro Thr Pro
           275                 280                 285
   Val Trp Phe His Val Leu Phe Tyr Leu Ala Val Gly Ile Met Phe Leu
       290                 295                 300
   Val Asn Thr Val Leu Trp Val Thr Ile Arg Lys Glu Leu Lys Arg Lys
   305                 310                 315                 320
   Lys Lys Trp Asp Leu Glu Ile Ser Leu Asp Ser Gly His Glu Lys Lys
                   325                 330                 335
   Val Ile Ser Ser Leu Gln Glu Asp Arg His Leu Glu Glu Glu Leu Lys
               340                 345                 350
   Cys Gln Glu Gln Lys Glu Glu Gln Leu Gln Glu Gly Val His Arg Lys
           355                 360                 365
   Glu Pro Gln Gly Ala Thr
       370
<210> 2
   <211> 291
   <212> PRT
   <213> Homo sapiens
<400> 2
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Ala Pro Pro
           35                  40                  45
   Lys Ala Val Leu Lys Leu Glu Pro Gln Trp Ile Asn Val Leu Gln Glu
       50                  55                  60
   Asp Ser Val Thr Leu Thr Cys Arg Gly Thr His Ser Pro Glu Ser Asp
   65                  70                  75                  80
   Ser Ile Gln Trp Phe His Asn Gly Asn Leu Ile Pro Thr His Thr Gln
                   85                  90                  95
   Pro Ser Tyr Arg Phe Lys Ala Asn Asn Asn Asp Ser Gly Glu Tyr Thr
               100                 105                 110
   Cys Gln Thr Gly Gln Thr Ser Leu Ser Asp Pro Val His Leu Thr Val
           115                 120                 125
   Leu Ser Glu Trp Leu Val Leu Gln Thr Pro His Leu Glu Phe Gln Glu
       130                 135                 140
   Gly Glu Thr Ile Val Leu Arg Cys His Ser Trp Lys Asp Lys Pro Leu
   145                 150                 155                 160
   Val Lys Val Thr Phe Phe Gln Asn Gly Lys Ser Lys Lys Phe Ser Arg
                   165                 170                 175
   Ser Asp Pro Asn Phe Ser Ile Pro Gln Ala Asn His Ser His Ser Gly
               180                 185                 190
   Asp Tyr His Cys Thr Gly Asn Ile Gly Tyr Thr Leu Tyr Ser Ser Lys
           195                 200                 205
   Pro Val Thr Ile Thr Val Gln Ala Pro Ser Ser Ser Pro Met Gly Ile
       210                 215                 220
   Ile Val Ala Val Val Thr Gly Ile Ala Val Ala Ala Ile Val Ala Ala
   225                 230                 235                 240
   Val Val Ala Leu Ile Tyr Cys Arg Lys Lys Arg Ile Ser Ala Asn Pro
                   245                 250                 255
   Thr Asn Pro Asp Glu Ala Asp Lys Val Gly Ala Glu Asn Thr Ile Thr
               260                 265                 270
   Tyr Ser Leu Leu Met His Pro Asp Ala Leu Glu Glu Pro Asp Asp Gln
           275                 280                 285
   Asn Arg Ile
       290
<210> 3
   <211> 280
   <212> PRT
   <213> Homo sapiens
<400> 3
   Met Trp Phe Leu Thr Thr Leu Leu Leu Trp Val Pro Val Asp Gly Gln
   1               5                   10                  15
   Val Asp Thr Thr Lys Ala Val Ile Thr Leu Gln Pro Pro Trp Val Ser
               20                  25                  30
   Val Phe Gln Glu Glu Thr Val Thr Leu His Cys Glu Val Leu His Leu
           35                  40                  45
   Pro Gly Ser Ser Ser Thr Gln Trp Phe Leu Asn Gly Thr Ala Thr Gln
       50                  55                  60
   Thr Ser Thr Pro Ser Tyr Arg Ile Thr Ser Ala Ser Val Asn Asp Ser
   65                  70                  75                  80
   Gly Glu Tyr Arg Cys Gln Arg Gly Leu Ser Gly Arg Ser Asp Pro Ile
                   85                  90                  95
   Gln Leu Glu Ile His Arg Gly Trp Leu Leu Leu Gln Val Ser Ser Arg
               100                 105                 110
   Val Phe Met Glu Gly Glu Pro Leu Ala Leu Arg Cys His Ala Trp Lys
           115                 120                 125
   Asp Lys Leu Val Tyr Asn Val Leu Tyr Tyr Arg Asn Gly Lys Ala Phe
       130                 135                 140
   Lys Phe Phe His Trp Asn Ser Asn Leu Thr Ile Leu Lys Thr Asn Ile
   145                 150                 155                 160
   Ser His Asn Gly Thr Tyr His Cys Ser Gly Met Gly Lys His Arg Tyr
   165 170 175
   Thr Ser Ala Gly Ile Ser Gln Tyr Thr Val Lys Gly Leu Gln Leu Pro
   180 185 190
   Thr Pro Val Trp Phe His Val Leu Phe Tyr Leu Ala Val Gly Ile Met
   195 200 205
   Phe Leu Val Asn Thr Val Leu Trp Val Thr Ile Arg Lys Glu Leu Lys
   210 215 220
   Arg Lys Lys Lys Trp Asn Leu Glu Ile Ser Leu Asp Ser Gly His Glu
   225 230 235 240
   Lys Lys Val Ile Ser Ser Leu Gln Glu Asp Arg His Leu Glu Glu Glu
   245 250 255
   Leu Lys Cys Gln Glu Gln Lys Glu Glu Gln Leu Gln Glu Gly Val His
   260 265 270
   Arg Lys Glu Pro Gln Gly Ala Thr
   275 280
<210> 4
   <211> 316
   <212> PRT
   <213> Homo sapiens
<400> 4
   Met Thr Met Glu Thr Gln Met Ser Gln Asn Val Cys Pro Arg Asn Leu
   1 5 10 15
   Trp Leu Leu Gln Pro Leu Thr Val Leu Leu Leu Leu Ala Ser Ala Asp
   20 25 30
   Ser Gln Ala Ala Pro Pro Lys Ala Val Leu Lys Leu Glu Pro Pro Trp
   35 40 45
   Ile Asn Val Leu Gln Glu Asp Ser Val Thr Leu Thr Cys Gln Gly Ala
   50 55 60
   Arg Ser Pro Glu Ser Asp Ser Ile Gln Trp Phe His Asn Gly Asn Leu
   65 70 75 80
   Ile Pro Thr His Thr Gln Pro Ser Tyr Arg Phe Lys Ala Asn Asn Asn
                   85                  90                  95
   Asp Ser Gly Glu Tyr Thr Cys Gln Thr Gly Gln Thr Ser Leu Ser Asp
               100                 105                 110
   Pro Val His Leu Thr Val Leu Ser Glu Trp Leu Val Leu Gln Thr Pro
           115                 120                 125
   His Leu Glu Phe Gln Glu Gly Glu Thr Ile Met Leu Arg Cys His Ser
       130                 135                 140
   Trp Lys Asp Lys Pro Leu Val Lys Val Thr Phe Phe Gln Asn Gly Lys
   145                 150                 155                 160
   Ser Gln Lys Phe Ser His Leu Asp Pro Thr Phe Ser Ile Pro Gln Ala
                   165                 170                 175
   Asn His Ser His Ser Gly Asp Tyr His Cys Thr Gly Asn Ile Gly Tyr
               180                 185                 190
   Thr Leu Phe Ser Ser Lys Pro Val Thr Ile Thr Val Gln Val Pro Ser
           195                 200                 205
   Met Gly Ser Ser Ser Pro Met Gly Ile Ile Val Ala Val Val Ile Ala
       210                 215                 220
   Thr Ala Val Ala Ala Ile Val Ala Ala Val Val Ala Leu Ile Tyr Cys
   225                 230                 235                 240
   Arg Lys Lys Arg Ile Ser Ala Asn Ser Thr Asp Pro Val Lys Ala Ala
                   245                 250                 255
   Gln Phe Glu Pro Pro Gly Arg Gln Met Ile Ala Ile Arg Lys Arg Gln
               260                 265                 270
   Leu Glu Glu Thr Asn Asn Asp Tyr Glu Thr Ala Asp Gly Gly Tyr Met
           275                 280                 285
   Thr Leu Asn Pro Arg Ala Pro Thr Asp Asp Asp Lys Asn Ile Tyr Leu
       290                 295                 300
   Thr Leu Pro Pro Asn Asp His Val Asn Ser Asn Asn
   305                 310                 315
<210> 5
   <211> 310
   <212> PRT
   <213> Homo sapiens
<400> 5
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Ala Pro Pro
           35                  40                  45
   Lys Ala Val Leu Lys Leu Glu Pro Gln Trp Ile Asn Val Leu Gln Glu
       50                  55                  60
   Asp Ser Val Thr Leu Thr Cys Arg Gly Thr His Ser Pro Glu Ser Asp
   65                  70                  75                  80
   Ser Ile Gln Trp Phe His Asn Gly Asn Leu Ile Pro Thr His Thr Gln
                   85                  90                  95
   Pro Ser Tyr Arg Phe Lys Ala Asn Asn Asn Asp Ser Gly Glu Tyr Thr
               100                 105                 110
   Cys Gln Thr Gly Gln Thr Ser Leu Ser Asp Pro Val His Leu Thr Val
           115                 120                 125
   Leu Ser Glu Trp Leu Val Leu Gln Thr Pro His Leu Glu Phe Gln Glu
       130                 135                 140
   Gly Glu Thr Ile Val Leu Arg Cys His Ser Trp Lys Asp Lys Pro Leu
   145                 150                 155                 160
   Val Lys Val Thr Phe Phe Gln Asn Gly Lys Ser Lys Lys Phe Ser Arg
                   165                 170                 175
   Ser Asp Pro Asn Phe Ser Ile Pro Gln Ala Asn His Ser His Ser Gly
               180                 185                 190
   Asp Tyr His Cys Thr Gly Asn Ile Gly Tyr Thr Leu Tyr Ser Ser Lys
           195                 200                 205
   Pro Val Thr Ile Thr Val Gln Ala Pro Ser Ser Ser Pro Met Gly Ile
       210                 215                 220
   Ile Val Ala Val Val Thr Gly Ile Ala Val Ala Ala Ile Val Ala Ala
   225 230 235 240
   Val Val Ala Leu Ile Tyr Cys Arg Lys Lys Arg Ile Ser Ala Leu Pro
   245 250 255
   Gly Tyr Pro Glu Cys Arg Glu Met Gly Glu Thr Leu Pro Glu Lys Pro
   260 265 270
   Ala Asn Pro Thr Asn Pro Asp Glu Ala Asp Lys Val Gly Ala Glu Asn
   275 280 285
   Thr Ile Thr Tyr Ser Leu Leu Met His Pro Asp Ala Leu Glu Glu Pro
   290 295 300
   Asp Asp Gln Asn Arg Ile
   305 310
<210> 6
   <211> 290
   <212> PRT
   <213> Homo sapiens
<400> 6
   Met Gly Ile Leu Ser Phe Leu Pro Val Leu Ala Thr Glu Ser Asp Trp
   1 5 10 15
   Ala Asp Cys Lys Ser Pro Gln Pro Trp Gly His Met Leu Leu Trp Thr
   20 25 30
   Ala Val Leu Phe Leu Ala Pro Val Ala Gly Thr Pro Ala Pro Pro Lys
   35 40 45
   Ala Val Leu Lys Leu Glu Pro Gln Trp Ile Asn Val Leu Gln Glu Asp
   50 55 60
   Ser Val Thr Leu Thr Cys Arg Gly Thr His Ser Pro Glu Ser Asp Ser
   65 70 75 80
   Ile Gln Trp Phe His Asn Gly Asn Leu Ile Pro Thr His Thr Gln Pro
   85 90 95
   Ser Tyr Arg Phe Lys Ala Asn Asn Asn Asp Ser Gly Glu Tyr Thr Cys
   100 105 110
   Gln Thr Gly Gln Thr Ser Leu Ser Asp Pro Val His Leu Thr Val Leu
               115                 120                 125
   Ser Glu Trp Leu Val Leu Gln Thr Pro His Leu Glu Phe Gln Glu Gly
       130                 135                 140
   Glu Thr Ile Val Leu Arg Cys His Ser Trp Lys Asp Lys Pro Leu Val
   145                 150                 155                 160
   Lys Val Thr Phe Phe Gln Asn Gly Lys Ser Lys Lys Phe Ser Arg Ser
                   165                 170                 175
   Asp Pro Asn Phe Ser Ile Pro Gln Ala Asn His Ser His Ser Gly Asp
               180                 185                 190
   Tyr His Cys Thr Gly Asn Ile Gly Tyr Thr Leu Tyr Ser Ser Lys Pro
           195                 200                 205
   Val Thr Ile Thr Val Gln Ala Pro Ser Ser Ser Pro Met Gly Ile Ile
       210                 215                 220
   Val Ala Val Val Thr Gly Ile Ala Val Ala Ala Ile Val Ala Ala Val
   225                 230                 235                 240
   Val Ala Leu Ile Tyr Cys Arg Lys Lys Arg Ile Ser Ala Asn Pro Thr
                   245                 250                 255
   Asn Pro Asp Glu Ala Asp Lys Val Gly Ala Glu Asn Thr Ile Thr Tyr
               260                 265                 270
   Ser Leu Leu Met His Pro Asp Ala Leu Glu Glu Pro Asp Asp Gln Asn
           275                 280                 285
   Arg Ile
       290
<210> 7
   <211> 291
   <212> PRT
   <213> Homo sapiens
<400> 7
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Ala Pro Pro
           35                  40                      45
   Lys Ala Val Leu Lys Leu Glu Pro Gln Trp Ile Asn Val Leu Gln Glu
       50                  55                  60
   Asp Ser Val Thr Leu Thr Cys Arg Gly Thr His Ser Pro Glu Ser Asp
   65                  70                  75                      80
   Ser Ile Gln Trp Phe His Asn Gly Asn Leu Ile Pro Thr His Thr Gln
                   85                  90                      95
   Pro Ser Tyr Arg Phe Lys Ala Asn Asn Asn Asp Ser Gly Glu Tyr Thr
               100                 105                     110
   Cys Gln Thr Gly Gln Thr Ser Leu Ser Asp Pro Val His Leu Thr Val
           115                 120                     125
   Leu Ser Glu Trp Leu Val Leu Gln Thr Pro His Leu Glu Phe Gln Glu
       130                 135                     140
   Gly Glu Thr Ile Val Leu Arg Cys His Ser Trp Lys Asp Lys Pro Leu
   145                 150                     155                     160
   Val Lys Val Thr Phe Phe Gln Asn Gly Lys Ser Lys Lys Phe Ser Arg
                   165                     170                     175
   Ser Asp Pro Asn Phe Ser Ile Pro Gln Ala Asn His Ser His Ser Gly
               180                     185                     190
   Asp Tyr His Cys Thr Gly Asn Ile Gly Tyr Thr Leu Tyr Ser Ser Lys
           195                     200                     205
   Pro Val Thr Ile Thr Val Gln Ala Pro Ser Ser Ser Pro Met Gly Ile
       210                     215                     220
   Ile Val Ala Val Val Thr Gly Ile Ala Val Ala Ala Ile Val Ala Ala
   225                     230                     235                     240
   Val Val Ala Leu Ile Tyr Cys Arg Lys Lys Arg Ile Ser Ala Asn Pro
                   245                     250                     255
   Thr Asn Pro Asp Glu Ala Asp Lys Val Gly Ala Glu Asn Thr Ile Thr
               260                     265                     270
   Tyr Ser Leu Leu Met His Pro Asp Ala Leu Glu Glu Pro Asp Asp Gln
           275                     280                     285
   Asn Arg Ile
       290
<210> 8
   <211> 309
   <212> PRT
   <213> Homo sapiens
<400> 8
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Pro Pro Lys
           35                  40                  45
   Ala Val Leu Lys Leu Glu Pro Gln Trp Ile Asn Val Leu Gln Glu Asp
       50                  55                  60
   Ser Val Thr Leu Thr Cys Arg Gly Thr His Ser Pro Glu Ser Asp Ser
   65                  70                  75                  80
   Ile Gln Trp Phe His Asn Gly Asn Leu Ile Pro Thr His Thr Gln Pro
                   85                  90                  95
   Ser Tyr Arg Phe Lys Ala Asn Asn Asn Asp Ser Gly Glu Tyr Thr Cys
               100                 105                 110
   Gln Thr Gly Gln Thr Ser Leu Ser Asp Pro Val His Leu Thr Val Leu
           115                 120                 125
   Ser Glu Trp Leu Val Leu Gln Thr Pro His Leu Glu Phe Gln Glu Gly
       130                 135                 140
   Glu Thr Ile Val Leu Arg Cys His Ser Trp Lys Asp Lys Pro Leu Val
   145                 150                 155                 160
   Lys Val Thr Phe Phe Gln Asn Gly Lys Ser Lys Lys Phe Ser Arg Ser
                   165                 170                 175
   Asp Pro Asn Phe Ser Ile Pro Gln Ala Asn His Ser His Ser Gly Asp
               180                 185                 190
   Tyr His Cys Thr Gly Asn Ile Gly Tyr Thr Leu Tyr Ser Ser Lys Pro
   195 200 205
   Val Thr Ile Thr Val Gln Ala Pro Ser Ser Ser Pro Met Gly Ile Ile
   210 215 220
   Val Ala Val Val Thr Gly Ile Ala Val Ala Ala Ile Val Ala Ala Val
   225 230 235 240
   Val Ala Leu Ile Tyr Cys Arg Lys Lys Arg Ile Ser Ala Leu Pro Gly
   245 250 255
   Tyr Pro Glu Cys Arg Glu Met Gly Glu Thr Leu Pro Glu Lys Pro Ala
   260 265 270
   Asn Pro Thr Asn Pro Asp Glu Ala Asp Lys Val Gly Ala Glu Asn Thr
   275 280 285
   Ile Thr Tyr Ser Leu Leu Met His Pro Asp Ala Leu Glu Glu Pro Asp
   290 295 300
   Asp Gln Asn Arg Ile
   305
<210> 9
   <211> 323
   <212> PRT
   <213> Homo sapiens
<400> 9
   Met Gly Ile Leu Ser Phe Leu Pro Val Leu Ala Thr Glu Ser Asp Trp
   1 5 10 15
   Ala Asp Cys Lys Ser Pro Gln Pro Trp Gly His Met Leu Leu Trp Thr
   20 25 30
   Ala Val Leu Phe Leu Ala Pro Val Ala Gly Thr Pro Ala Ala Pro Pro
   35 40 45
   Lys Ala Val Leu Lys Leu Glu Pro Gln Trp Ile Asn Val Leu Gln Glu
   50 55 60
   Asp Ser Val Thr Leu Thr Cys Arg Gly Thr His Ser Pro Glu Ser Asp
   65 70 75 80
   Ser Ile Pro Trp Phe His Asn Gly Asn Leu Ile Pro Thr His Thr Gln
                   85                  90                  95
   Pro Ser Tyr Arg Phe Lys Ala Asn Asn Asn Asp Ser Gly Glu Tyr Thr
               100                 105                 110
   Cys Gln Thr Gly Gln Thr Ser Leu Ser Asp Pro Val His Leu Thr Val
           115                 120                 125
   Leu Ser Glu Trp Leu Val Leu Gln Thr Pro His Leu Glu Phe Gln Glu
       130                 135                 140
   Gly Glu Thr Ile Val Leu Arg Cys His Ser Trp Lys Asp Lys Pro Leu
   145                 150                 155                 160
   Val Lys Val Thr Phe Phe Gln Asn Gly Lys Ser Lys Lys Phe Ser Arg
                   165                 170                 175
   Ser Asp Pro Asn Phe Ser Ile Pro Gln Ala Asn His Ser His Ser Gly
               180                 185                 190
   Asp Tyr His Cys Thr Gly Asn Ile Gly Tyr Thr Leu Tyr Ser Ser Lys
           195                 200                 205
   Pro Val Thr Ile Thr Val Gln Ala Pro Ser Ser Ser Pro Met Gly Ile
       210                 215                 220
   Ile Val Ala Val Val Thr Gly Ile Ala Val Ala Ala Ile Val Ala Ala
   225                 230                 235                 240
   Val Val Ala Leu Ile Tyr Cys Arg Lys Lys Arg Ile Ser Ala Asn Ser
                   245                 250                 255
   Thr Asp Pro Val Lys Ala Ala Gln Phe Glu Pro Pro Gly Arg Gln Met
               260                 265                 270
   Ile Ala Ile Arg Lys Arg Gln Pro Glu Glu Thr Asn Asn Asp Tyr Glu
           275                 280                 285
   Thr Ala Asp Gly Gly Tyr Met Thr Leu Asn Pro Arg Ala Pro Thr Asp
       290                 295                 300
   Asp Asp Lys Asn Ile Tyr Leu Thr Leu Pro Pro Asn Asp His Val Asn
   305                 310                 315                 320
   Ser Asn Asn
<210> 10
   <211> 276
   <212> PRT
   <213> Homo sapiens
<400> 10
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Ala Pro Pro
           35                  40                  45
   Lys Ala Val Leu Lys Leu Glu Pro Gln Trp Ile Asn Val Leu Gln Glu
       50                  55                  60
   Asp Ser Val Thr Leu Thr Cys Arg Gly Thr His Ser Pro Glu Ser Asp
   65                  70                  75                  80
   Ser Ile Pro Trp Phe His Asn Gly Asn Leu Ile Pro Thr His Thr Gln
                   85                  90                  95
   Pro Ser Tyr Arg Phe Lys Ala Asn Asn Asn Asp Ser Gly Glu Tyr Thr
               100                 105                 110
   Cys Gln Thr Gly Gln Thr Ser Leu Ser Asp Pro Val His Leu Thr Val
           115                 120                 125
   Leu Ser Glu Trp Leu Val Leu Gln Thr Pro His Leu Glu Phe Gln Glu
       130                 135                 140
   Gly Glu Thr Ile Val Leu Arg Cys His Ser Trp Lys Asp Lys Pro Leu
   145                 150                 155                 160
   Val Lys Val Thr Phe Phe Gln Asn Gly Lys Ser Lys Lys Phe Ser Arg
                   165                 170                 175
   Ser Asp Pro Asn Phe Ser Ile Pro Gln Ala Asn His Ser His Ser Gly
               180                 185                 190
   Asp Tyr His Cys Thr Gly Asn Ile Gly Tyr Thr Leu Tyr Ser Ser Lys
           195                 200                 205
   Pro Val Thr Ile Thr Val Gln Ala Pro Ser Ser Ser Pro Met Gly Ile
       210                 215                 220
   Ile Val Ala Val Val Thr Gly Ile Ala Val Ala Ala Ile Val Ala Ala
   225                 230                 235                 240
   Val Val Ala Leu Ile Tyr Cys Arg Lys Lys Arg Ile Ser Ala Asn Ser
                   245                 250                 255
   Thr Asp Pro Val Lys Ala Ala Gln Phe Glu Met Leu Ser Cys Thr His
               260                 265                 270
   Leu Asp Val Lys
           275
<210> 11
   <211> 267
   <212> PRT
   <213> Homo sapiens
<400> 11
   Met Gly Ile Leu Ser Phe Leu Pro Val Leu Ala Thr Glu Ser Asp Trp
   1               5                   10                  15
   Ala Asp Cys Lys Ser Pro Gln Pro Trp Gly His Met Leu Leu Trp Thr
               20                  25                  30
   Ala Val Leu Phe Leu Ala Pro Val Ala Gly Thr Pro Ala Ala Pro Pro
           35                  40                  45
   Lys Ala Val Leu Lys Leu Glu Pro Gln Trp Ile Asn Val Leu Gln Glu
       50                  55                  60
   Asp Ser Val Thr Leu Thr Cys Arg Gly Thr His Ser Pro Glu Ser Asp
   65                  70                  75                  80
   Ser Ile Pro Trp Phe His Asn Gly Asn Leu Ile Pro Thr His Thr Gln
                   85                  90                  95
   Pro Ser Tyr Arg Phe Lys Ala Asn Asn Asn Asp Ser Gly Glu Tyr Thr
               100                 105                 110
   Cys Gln Thr Gly Gln Thr Ser Leu Ser Asp Pro Val His Leu Thr Val
           115                 120                 125
   Leu Ser Glu Trp Leu Val Leu Gln Thr Pro His Leu Glu Phe Gln Glu
       130                 135                 140
   Gly Glu Thr Ile Val Leu Arg Cys His Ser Trp Lys Asp Lys Pro Leu
   145                 150                 155                 160
   Val Lys Val Thr Phe Phe Gln Asn Gly Lys Ser Lys Lys Phe Ser Arg
                   165                 170                 175
   Ser Asp Pro Asn Phe Ser Ile Pro Gln Ala Asn His Ser His Ser Gly
               180                 185                 190
   Asp Tyr His Cys Thr Gly Asn Ile Gly Tyr Thr Leu Tyr Ser Ser Lys
           195                 200                 205
   Pro Val Thr Ile Thr Val Gln Ala Pro Ser Ser Ser Pro Met Gly Ile
       210                 215                 220
   Ile Val Ala Val Val Thr Gly Ile Ala Val Ala Ala Ile Val Ala Ala
   225                 230                 235                 240
   Val Val Ala Leu Ile Tyr Cys Arg Lys Lys Arg Ile Ser Ala Thr Trp
                   245                 250                 255
   Thr Ser Asn Asp Cys His Gln Lys Glu Thr Thr
               260                 265
<210> 12
   <211> 254
   <212> PRT
   <213> Homo sapiens
<400> 12
<210> 13
   <211> 85
   <212> PRT
   <213> Homo sapiens
<400> 13
   Asp Leu Pro Lys Ala Val Val Phe Leu Glu Pro Gln Trp Tyr Ser Val
   1 5 10 15
   Leu Glu Lys Asp Ser Val Thr Leu Lys Cys Gln Gly Ala Tyr Ser Pro
   20 25 30
   Glu Asp Asn Ser Thr Gln Trp Phe His Asn Glu Ser Leu Ile Ser Ser
                   35                  40                  45
   Gln Ala Ser Ser Tyr Phe Ile Asp Ala Ala Thr Val Asn Asp Ser Gly
       50                  55                  60
   Glu Tyr Arg Cys Gln Thr Asn Leu Ser Thr Leu Ser Asp Pro Val Gln
   65                  70                  75                  80
   Leu Glu Val His Ile
                   85
<210> 14
   <211> 1125
   <212> DNA
   <213> Homo sapiens
<400> 14
   atgtggttct tgacaactct gctcctttgg gttccagttg atgggcaagt ggacaccaca      60
   aaggcagtga tcactttgca gcctccatgg gtcagcgtgt tccaagagga aaccgtaacc     120
   ttgcactgtg aggtgctcca tctgcctggg agcagctcta cacagtggtt tctcaatggc     180
   acagccactc agacctcgac ccccagctac agaatcacct ctgccagtgt caatgacagt     240
   ggtgaataca ggtgccagag aggtctctca gggcgaagtg accccataca gctggaaatc     300
   cacagaggct ggctactact gcaggtctcc agcagagtct tcacggaagg agaacctctg     360
   gccttgaggt gtcatgcgtg gaaggataag ctggtgtaca atgtgcttta ctatcgaaat     420
   ggcaaagcct ttaagttttt ccactggaat tctaacctca ccattctgaa aaccaacata     480
   agtcacaatg gcacctacca ttgctcaggc atgggaaagc atcgctacac atcagcagga     540
   atatctgtca ctgtgaaaga gctatttcca gctccagtgc tgaatgcatc tgtgacatcc     600
   ccactcctgg aggggaatct ggtcaccctg agctgtgaaa caaagttgct cttgcagagg     660
   cctggtttgc agctttactt ctccttctac atgggcagca agaccctgcg aggcaggaac     720
   acatcctctg aataccaaat actaactgct agaagagaag actctgggtt atactggtgc     780
   gaggctgcca cagaggatgg aaatgtcctt aagcgcagcc ctgagttgga gcttcaagtg     840
   cttggcctcc agttaccaac tcctgtctgg tttcatgtcc ttttctatct ggcagtggga     900
   ataatgtttt tagtgaacac tgttctctgg gtgacaatac gtaaagaact gaaaagaaag     960
   aaaaagtggg atttagaaat ctctttggat tctggtcatg agaagaaggt aatttccagc    1020
   cttcaagaag acagacattt agaagaagag ctgaaatgtc aggaacaaaa agaagaacag    1080
   ctgcaggaag gggtgcaccg gaaggagccc caggggccca cgtag                     1125
<210> 15
   <211> 567
   <212> DNA
   <213> Homo sapiens
<400> 15
   atgtggttct tgacaactct gctcctttgg ggctggctac tactgcaggt ctccagcaga      60
   gtcttcatgg aaggagaacc tctggccttg aggtgtcatg cgtggaagga taagctggtg     120
   tacaatgtgc tttactatcg aaatggcaaa gcctttaagt ttttccactg gaattctaac     180
   ctcaccattc tgaaaaccaa cataagtcac aatggcacct accattgctc aggcatggga     240
   aagcatcgct acacatcagc aggaatatca caatacactg tgaaaggcct ccagttacca     300
   actcctgtct ggtttcatgt ccttttctat ctggcagtgg gaataatgtt tttagtgaac     360
   actgttctct gggtgacaat acgtaaagaa ctgaaaagaa agaaaaagtg gaatttagaa     420
   atctctttgg attctggtca tgagaagaag gtaatttcca gccttcaaga agacagacat     480
   ttagaagaag agctgaaatg tcaggaacaa aaagaagaac agctgcagga aggggtgcac     540
   cggaaggagc cccagggggc cacgtag                                         567
<210> 16
   <211> 843
   <212> DNA
   <213> Homo sapiens
<400> 16
   atgtggttct tgacaactct gctcctttgg gttccagttg atgggcaagt ggacaccaca      60
   aaggcagtga tcactttgca gcctccatgg gtcagcgtgt tccaagagga aaccgtaacc     120
   ttgcactgtg aggtgctcca tctgcctggg agcagctcca cacagtggtt tctcaatggc     180
   acagccactc agacctcgac ccccagctac agaatcacct ctgccagtgt caatgacagt     240
   ggtgaataca ggtgccagag aggtctctca gggcgaagtg accccataca gctggaaatc     300
   cacagaggct ggctactact gcaggtctcc agcagagtct tcatggaagg agaacctctg     360
   gccttgaggt gtcatgcgtg gaaggataag ctggtgtaca atgtgcttta ctatcgaaat     420
   ggcaaagcct ttaagttttt ccactggaat tctaacctca ccattctgaa aaccaacata     480
   agtcacaatg gcacctacca ttgctcaggc atgggaaagc atcgctacac atcagcagga     540
   atatcacaat acactgtgaa aggcctccag ttaccaactc ctgtctggtt tcatgtcctt     600
   ttctatctgg cagtgggaat aatgttttta gtgaacactg ttctctgggt gacaatacgt     660
   aaagaactga aaagaaagaa aaagtggaat ttagaaatct ctttggattc tggtcatgag     720
   aagaaggtaa tttccagcct tcaagaagac agacatttag aagaagagct gaaatgtcag     780
   gaacaaaaag aagaacagct gcaggaaggg gtgcaccgga aggagcccca gggggccacg     840
   tag                                                                   843
<210> 17
   <211> 951
   <212> DNA
   <213> Homo sapiens
<400> 17
   atgactatgg agacccaaat gtctcagaat gtatgtccca gaaacctgtg gctgcttcaa 60
   ccattgacag ttttgctgct gctggcttct gcagacagtc aagctgctcc cccaaaggct 120
   gtgctgaaac ttgagccccc gtggatcaac gtgctccagg aggactctgt gactctgaca 180
   tgccaggggg ctcgcagccc tgagagcgac tccattcagt ggttccacaa tgggaatctc 240
   attcccaccc acacgcagcc cagctacagg ttcaaggcca acaacaatga cagcggggag 300
   tacacgtgcc agactggcca gaccagcctc agcgaccctg tgcatctgac tgtgctttcc 360
   gaatggctgg tgctccagac ccctcacctg gagttccagg agggagaaac catcatgctg 420
   aggtgccaca gctggaagga caagcctctg gtcaaggtca cattcttcca gaatggaaaa 480
   tcccagaaat tctcccattt ggatcccacc ttctccatcc cacaagcaaa ccacagtcac 540
   agtggtgatt accactgcac aggaaacata ggctacacgc tgttctcatc caagcctgtg 600
   accatcactg tccaagtgcc cagcatgggc agctcttcac caatggggat cattgtggct 660
   gtggtcattg cgactgctgt agcagccatt gttgctgctg tagtggcctt gatctactgc 720
   aggaaaaagc ggatttcagc caattccact gatcctgtga aggctgccca atttgagcca 780
   cctggacgtc aaatgattgc catcagaaag agacaacttg aagaaaccaa caatgactat 840
   gaaacagctg acggcggcta catgactctg aaccccaggg cacctactga cgatgataaa 900
   aacatctacc tgactcttcc tcccaacgac catgtcaaca gtaataacta a 951
<210> 18
   <211> 933
   <212> DNA
   <213> Homo sapiens
<400> 18
   atgggaatcc tgtcattctt acctgtcctt gccactgaga gtgactgggc tgactgcaag 60
   tccccccagc cttggggtca tatgcttctg tggacagctg tgctattcct ggctcctgtt 120
   gctgggacac ctgcagctcc cccaaaggct gtgctgaaac tcgagcccca gtggatcaac 180
   gtgctccagg aggactctgt gactctgaca tgccggggga ctcacagccc tgagagcgac 240
   tccattcagt ggttccacaa tgggaatctc attcccaccc acacgcagcc cagctacagg 300
   ttcaaggcca acaacaatga cagcggggag tacacgtgcc agactggcca gaccagcctc 360
   agcgaccctg tgcatctgac tgtgctttct gagtggctgg tgctccagac ccctcacctg 420
   gagttccagg agggagaaac catcgtgctg aggtgccaca gctggaagga caagcctctg 480
   gtcaaggtca cattcttcca gaatggaaaa tccaagaaat tttcccgttc ggatcccaac 540
   ttctccatcc cacaagcaaa ccacagtcac agtggtgatt accactgcac aggaaacata 600
   ggctacacgc tgtactcatc caagcctgtg accatcactg tccaagctcc cagctcttca 660
   ccgatgggga tcattgtggc tgtggtcact gggattgctg tagcggccat tgttgctgct 720
   gtagtggcct tgatctactg caggaaaaag cggatttcag ctctcccagg ataccctgag 780
   tgcagggaaa tgggagagac cctccctgag aaaccagcca atcccactaa tcctgatgag 840
   gctgacaaag ttggggctga gaacacaatc acctattcac ttctcatgca cccggatgct 900
   ctggaagagc ctgatgacca gaaccgtatt tag 933
<210> 19
   <211> 798
   <212> DNA
   <213> Homo sapiens
<400> 19
   tctagcgagg tgacagcgta gaaccagaga atttgtttgc cctctagggt agaatccgcc 60
   aagctttgag agaaggctgt gactgctgtg ctctgggcgc cagctcgctc cagggagtgg 120
   tgggaatcct gtcattctta cctgtccttg ccactgagag tgactgggct gactgcaagt 180
   ccccccagcc ttggggtcat atgcttctgt ggacagctgt gctattcctg gctcctgttg 240
   ctgggacacc tgcagctccc ccaaaggctg tgctgaaact cgagccccag tggatcaacg 300
   tgctccagga ggactctgtg actctgacat gccgggggac tcacagccct gagagcgact 360
   ccattcagtg gttccacaat gggaatctca ttcccaccca cacgcagccc agctacaggt 420
   tcaaggccaa caacaatgac agcggggagt acacgtgcca gactggccag accagcctca 480
   gcgaccctgt gcatctgact gtgctttctg agtggctggt gctccagacc cctcacctgg 540
   agttccagga gggagaaacc atcgtgctga ggtgccacag ctggaaagac aagcctctgg 600
   tcaaggtcac attcttccag aatggaaaat ccaagaaatt ttcccgttcg gatcccaact 660
   tctccatccc acaagcaaac cacagtccca gtgggtgatt accactgcac aggaaaaaat 720
   agggctacac cctgtactca tccaagccct gtggaccatc actgttcaaa gctccccaac 780
   ttcttcacgg atggggga 798
<210> 20
   <211> 876
   <212> DNA
   <213> Homo sapiens
<400> 20
   atgggaatcc tgtcattctt acctgtcctt gccactgaga gtgactgggc tgactgcaag 60
   tccccccagc cttggggtca tatgcttctg tggacagctg tgctattcct ggctcctgtt 120
   gctgggacac ctgcagctcc cccaaaggct gtgctgaaac tcgagcccca gtggatcaac 180
   gtgctccagg aggactctgt gactctgaca tgccggggga ctcacagccc tgagagcgac 240
   tccattcagt ggttccacaa tgggaatctc attcccaccc acacgcagcc cagctacagg 300
   ttcaaggcca acaacaatga cagcggggag tacacgtgcc agactggcca gaccagcctc 360
   agcgaccctg tgcatctgac tgtgctttct gagtggctgg tgctccagac ccctcacctg 420
   gagttccagg agggagaaac catcgtgctg aggtgccaca gctggaagga caagcctctg 480
   gtcaaggtca cattcttcca gaatggaaaa tccaagaaat tttcccgttc ggatcccaac 540
   ttctccatcc cacaagcaaa ccacagtcac agtggtgatt accactgcac aggaaacata 600
   ggctacacgc tgtactcatc caagcctgtg accatcactg tccaagctcc cagctcttca 660
   ccgatgggga tcattgtggc tgtggtcact gggattgctg tagcggccat tgttgctgct 720
   gtagtggcct tgatctactg caggaaaaag cggatttcag ccaatcccac taatcctgat 780
   gaggctgaca aagttggggc tgagaacaca atcacctatt cacttctcat gcacccggat 840
   gctctggaag agcctgatga ccagaaccgt atttag 876
<210> 21
   <211> 930
   <212> DNA
   <213> Homo sapiens
<400> 21
   atgggaatcc tgtcattctt acctgtcctt gccactgaga gtgactgggc tgactgcaag 60
   tccccccagc cttggggtca tatgcttctg tggacagctg tgctattcct ggctcctgtt 120
   gctgggacac ctgctccccc aaaggctgtg ctgaaactcg agccccagtg gatcaacgtg 180
   ctccaggagg actctgtgac tctgacatgc cgggggactc acagccctga gagcgactcc 240
   attcagtggt tccacaatgg gaatctcatt cccacccaca cgcagcccag ctacaggttc 300
   aaggccaaca acaatgacag cggggagtac acgtgccaga ctggccagac cagcctcagc 360
   gaccctgtgc atctgactgt gctttctgag tggctggtgc tccagacccc tcacctggag 420
   ttccaggagg gagaaaccat cgtgctgagg tgccacagct ggaaggacaa gcctctggtc 480
   aaggtcacat tcttccagaa tggaaaatcc aagaaatttt cccgttcgga tcccaacttc 540
   tccatcccac aagcaaacca cagtcacagt ggtgattacc actgcacagg aaacataggc 600
   tacacgctgt actcatccaa gcctgtgacc atcactgtcc aagctcccag ctcttcaccg 660
   atggggatca ttgtggctgt ggtcactggg attgctgtag cggccattgt tgctgctgta 720
   gtggccttga tctactgcag gaaaaagcgg atttcagctc tcccaggata ccctgagtgc 780
   agggaaatgg gagagaccct ccctgagaaa ccagccaatc ccactaatcc tgatgaggct 840
   gacaaagttg gggctgagaa cacaatcacc tattcacttc tcatgcaccc ggatgctctg 900
   gaagagcctg atgaccagaa ccgtatttag 930
<210> 22
   <211> 972
   <212> DNA
   <213> Homo sapiens
<400> 22
   atgggaatcc tgtcattctt acctgtcctt gccactgaga gtgactgggc tgactgcaag      60
   tccccccagc cttggggtca tatgcttctg tggacagctg tgctattcct ggctcctgtt     120
   gctgggacac ctgcagctcc cccaaaggct gtgctgaaac tcgagcccca gtggatcaac     180
   gtgctccaag aggactctgt gactctgaca tgccggggga ctcacagccc tgagagcgac      240
   tccattccgt ggttccacaa tgggaatctc attcccaccc acacgcagcc cagctacagg     300
   ttcaaggcca acaacaatga cagcggggag tacacgtgcc agactggcca gaccagcctc     360
   agcgaccctg tgcatctgac tgtgctttct gagtggctgg tgctccagac ccctcacctg     420
   gagttccagg agggagaaac catcgtgctg aggtgccaca gctggaagga caagcctctg     480
   gtcaaggtca cattcttcca gaatggaaaa tccaagaaat tttcccgttc ggatcccaac     540
   ttctccatcc cacaagcaaa ccacagtcac agtggtgatt accactgcac aggaaacata     600
   ggctacacgc tgtactcatc caagcctgtg accatcactg tccaagctcc cagctcttca     660
   ccgatgggga tcattgtggc tgtggtcact gggattgctg tagcggccat tgttgctgct     720
   gtagtggcct tgatctactg caggaaaaag cggatttcag ccaattccac tgatcctgtg     780
   aaggctgccc aatttgagcc acctggacgt caaatgattg ccatcagaaa gagacaacct     840
   gaagaaacca acaatgacta tgaaacagct gacggcggct acatgactct gaaccccagg     900
   gcacctactg acgatgataa aaacatctac ctgactcttc ctcccaacga ccatgtcaac     960
   agtaataact aa                                                         972
<210> 23
   <211> 831
   <212> DNA
   <213> Homo sapiens
<400> 23
   atgggaatcc tgtcattctt acctgtcctt gccactgaga gtgactgggc tgactgcaag      60
   tccccccagc cttggggtca tatgcttctg tggacagctg tgctattcct ggctcctgtt     120
   gctgggacac ctgcagctcc cccaaaggct gtgctgaaac tcgagcccca gtggatcaac     180
   gtgctccaag aggactctgt gactctgaca tgccggggga ctcacagccc tgagagcgac 240
   tccattccgt ggttccacaa tgggaatctc attcccaccc acacgcagcc cagctacagg 300
   ttcaaggcca acaacaatga cagcggggag tacacgtgcc agactggcca gaccagcctc 360
   agcgaccctg tgcatctgac tgtgctttct gagtggctgg tgctccagac ccctcacctg 420
   gagttccagg agggagaaac catcgtgctg aggtgccaca gctggaagga caagcctctg 480
   gtcaaggtca cattcttcca gaatggaaaa tccaagaaat tttcccgttc ggatcccaac 540
   ttctccatcc cacaagcaaa ccacagtcac agtggtgatt accactgcac aggaaacata 600
   ggctacacgc tgtactcatc caagcctgtg accatcactg tccaagctcc cagctcttca 660
   ccgatgggga tcattgtggc tgtggtcact gggattgctg tagcggccat tgttgctgct 720
   gtagtggcct tgatctactg caggaaaaag cggatttcag ccaattccac tgatcctgtg 780
   aaggctgccc aatttgagat gctttcctgc acccacctgg acgtcaaatg a 831
<210> 24
   <211> 804
   <212> DNA
   <213> Homo sapiens
<400> 24
   atgggaatcc tgtcattctt acctgtcctt gccactgaga gtgactgggc tgactgcaag 60
   tccccccagc cttggggtca tatgcttctg tggacagctg tgctattcct ggctcctgtt 120
   gctgggacac ctgcagctcc cccaaaggct gtgctgaaac tcgagcccca gtggatcaac 180
   gtgctccaag aggactctgt gactctgaca tgccggggga ctcacagccc tgagagcgac 240
   tccattccgt ggttccacaa tgggaatctc attcccaccc acacgcagcc cagctacagg 300
   ttcaaggcca acaacaatga cagcggggag tacacgtgcc agactggcca gaccagcctc 360
   agcgaccctg tgcatctgac tgtgctttct gagtggctgg tgctccagac ccctcacctg 420
   gagttccagg agggagaaac catcgtgctg aggtgccaca gctggaagga caagcctctg 480
   gtcaaggtca cattcttcca gaatggaaaa tccaagaaat tttcccgttc ggatcccaac 540
   ttctccatcc cacaagcaaa ccacagtcac agtggtgatt accactgcac aggaaacata 600
   ggctacacgc tgtactcatc caagcctgtg accatcactg tccaagctcc cagctcttca 660
   ccgatgggga tcattgtggc tgtggtcact gggattgctg tagcggccat tgttgctgct 720
   gtagtggcct tgatctactg caggaaaaag cggatttcag ccacctggac gtcaaatgat 780
   tgccatcaga aagagacaac ctga 804
<210> 25
   <211> 873
   <212> DNA
   <213> Homo sapiens
<400> 25
   atgggtggag gggctgggga aaggctgttt acttcctcct gtctagtcgg tttggtccct      60
   ttagggctcc ggatatcttt ggtgacttgt ccactccagt gtggcatcat gtggcagctg     120
   ctcctcccaa ctgctctgct acttctagtt tcagctggca tgcggactga agatctccca     180
   aaggctgtgg tgttcctgga gcctcaatgg tacagggtgc tcgagaagga cagtgtgact     240
   ctgaagtgcc agggagccta ctcccctgag gacaattcca cacagtggtt tcacaatgag     300
   agcctcatct caagccaggc ctcgagctac ttcattgacg ctgccacagt cgacgacagt     360
   ggagagtaca ggtgccagac aaacctctcc accctcagtg acccggtgca gctagaagtc     420
   catatcggct ggctgttgct ccaggcccct cggtgggtgt tcaaggagga agaccctatt     480
   cacctgaggt gtcacagctg gaagaacact gctctgcata aggtcacata tttacagaat     540
   ggcaaaggca ggaagtattt tcatcataat tctgacttct acattccaaa agccacactc     600
   aaagacagcg gctcctactt ctgcaggggg ctttttggga gtaaaaatgt gtcttcagag     660
   actgtgaaca tcaccatcac tcaaggtttg gcagtgtcaa ccatctcatc attctttcca     720
   cctgggtacc aagtctcttt ctgcttggtg atggtactcc tttttgcagt ggacacagga     780
   ctatatttct ctgtgaagac aaacattcga agctcaacaa gagactggaa ggaccataaa     840
   tttaaatgga gaaaggaccc tcaagacaaa tga                                 873
<210> 26
   <211> 702
   <212> DNA
   <213> Homo sapiens
<400> 26
   atgtggcagc tgctcctccc aactgctctg ctacttctag tttcagctgg catgcggact     60
   gaagatctcc caaaggctgt ggtgttcctg gagcctcaat ggtacagcgt gcttgagaag    120
   gacagtgtga ctctgaagtg ccagggagcc tactcccctg aggacaattc cacacagtgg    180
   tttcacaatg agaacctcat ctcaagccag gcctcgagct acttcattga cgctgccaca    240
   gtcaacgaca gtggagagta caggtgccag acaaacctct ccaccctcag tgacccggtg    300
   cagctagaag tccatatcgg ctggctgttg ctccaggccc ctcggtgggt gttcaaggag    360
   gaagacccta ttcacctgag gtgtcacagc tggaagaaca ctgctctgca taaggtcaca    420
   tatttacaga atggcaaaga caggaagtat tttcatcata attctgactt ccacattcca    480
   aaagccacac tcaaagatag cggctcctac ttctgcaggg ggcttgttgg gagtaaaaat    540
   gtgtcttcag agactgtgaa catcaccatc actcaaggtt tggcagtgtc aaccatctca    600
   tcattctctc cacctgggta ccaagtctct ttctgcttgg tgatggtact cctttttgca    660
   gtggacacag gactatattt ctctgtgaag acaaacattt ga                        702